(19) Europäisches Patentamt
European Patent Office
Office européen des brevets

(11) Publication number: **0 517 041 A1**

(12) **EUROPEAN PATENT APPLICATION**

(21) Application number: **92108580.9**

(22) Date of filing: **21.05.92**

(51) Int. Cl.[5]: **C07D 501/46**, C07D 285/08, A61K 31/545

(30) Priority: **07.06.91 GB 9112248**
**29.11.91 GB 9125668**
**23.12.91 GB 9127278**

(43) Date of publication of application:
**09.12.92 Bulletin 92/50**

(84) Designated Contracting States:
**AT BE CH DE DK ES FR GB GR IT LI LU NL PT SE**

(71) Applicant: **FUJISAWA PHARMACEUTICAL CO., LTD.**
**4-7, Doshomachi 3-chome Chuo-ku**
**Osaka-shi Osaka 541(JP)**

(72) Inventor: **Kawabata, Kohji**
**1-72, Maruyamadai 2-chome**
**Kawanishi-shi, Hyogo 666-01(JP)**
Inventor: **Okuda, Shinya**
**2-2-10, Midorigaoka**
**Ikeda-shi, Osaka 563(JP)**
Inventor: **Yoshida, Yoshiki**
**6-C-704, Satsukigaokahigashi**
**Suita-shi, Osaka 565(JP)**
Inventor: **Eikyu, Yoshiteru**
**2-2-10, Midorigaoka**
**Ikeda-shi, Osaka 563(JP)**
Inventor: **Ogawa, Yasuhiro**
**2-2-10, Midorigaoka**
**Ikeda-shi, Osaka 563(JP)**
Inventor: **Yamanaka, Hideaki**
**77-10, Kuzuhanakanoshiba 2-chome**
**Hirakata-shi, Osaka 573(JP)**
Inventor: **Sakane, Kazuo**
**15, Azayamagata, Mino**
**Kawanishi-shi, Hyogo 666-01(JP)**

(74) Representative: **Türk, Gille, Hrabal, Leifert**
**Brucknerstrasse 20**
**W-4000 Düsseldorf 13(DE)**

(54) **New Cephem Compounds.**

(57) A compound of the formula :

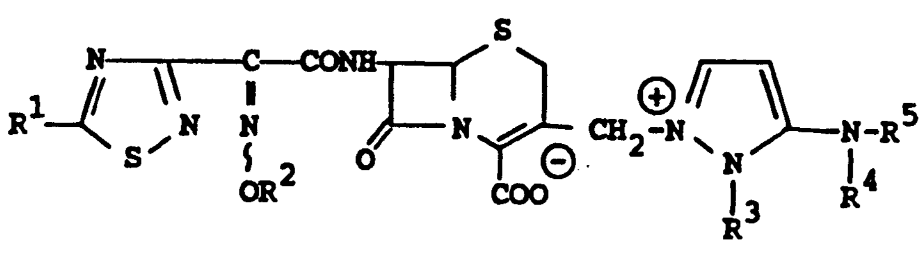

wherein

| | |
|---|---|
| $R^1$ is | amino or protected amino, |
| $R^2$ is | lower alkyl substituted with 1 to 6 halogen, |
| $R^3$ is | hydroxy(lower)alkyl, protected hydroxy(lower)alkyl or halo(lower)alkyl and |
| $R^4$ is | hydrogen, or |
| $R^3$ and $R^4$ are | linked together to form lower alkylene, and |
| $R^5$ is | hydrogen or an amino protective group, |

and a pharmaceutically acceptable salt thereof, processes for their preparation and pharmaceutical compositions

EP 0 517 041 A1

comprising them. The invention also relates to intermediate products of the formula

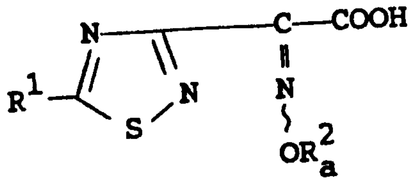

and processes for their preparation.

The present invention relates to new cephem compounds and pharmaceutically acceptable salts thereof. More particularly, it relates to new cephem compounds and pharmaceutically acceptable salts thereof, which have antimicrobial activities, to processes for preparation thereof, to pharmaceutical composition comprising the same, and to a method for treating infectious diseases in human being and animals.

Accordingly, one object of the present invention is to provide the cephem compounds and pharmaceutically acceptable salts thereof, which are highly active against a number of pathogenic microorganisms.

Another object of the present invention is to provide processes for the preparation of the cephem compounds and salts thereof.

A further object of the present invention is to provide pharmaceutical composition comprising, as an active ingredient, said cephem compounds or their pharmaceutically acceptable salts.

Still further object of the present invention is to provide a method for treating infectious diseases caused by pathogenic microorganisms, which comprises administering said cephem compounds to infected human being or animals.

The object cephem compounds of the present invention are novel and can be represented by the following general formula (I) :

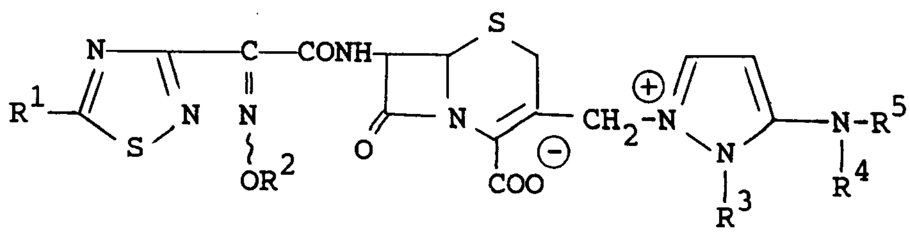

(I)

wherein

| | |
|---|---|
| $R^1$ is | amino or protected amino, |
| $R^2$ is | lower alkyl substituted with 1 to 6 halogen, |
| $R^3$ is | hydroxy(lower)alkyl, protected hydroxy(lower)alkyl or halo(lower)alkyl and |
| $R^4$ is | hydrogen, or |
| $R^3$ and $R^4$ are | linked together to form lower alkylene, and |
| $R^5$ is | hydrogen or an amino protective group. |

The object compound (I) of the present invention can be prepared by the following processes.

Process (1)

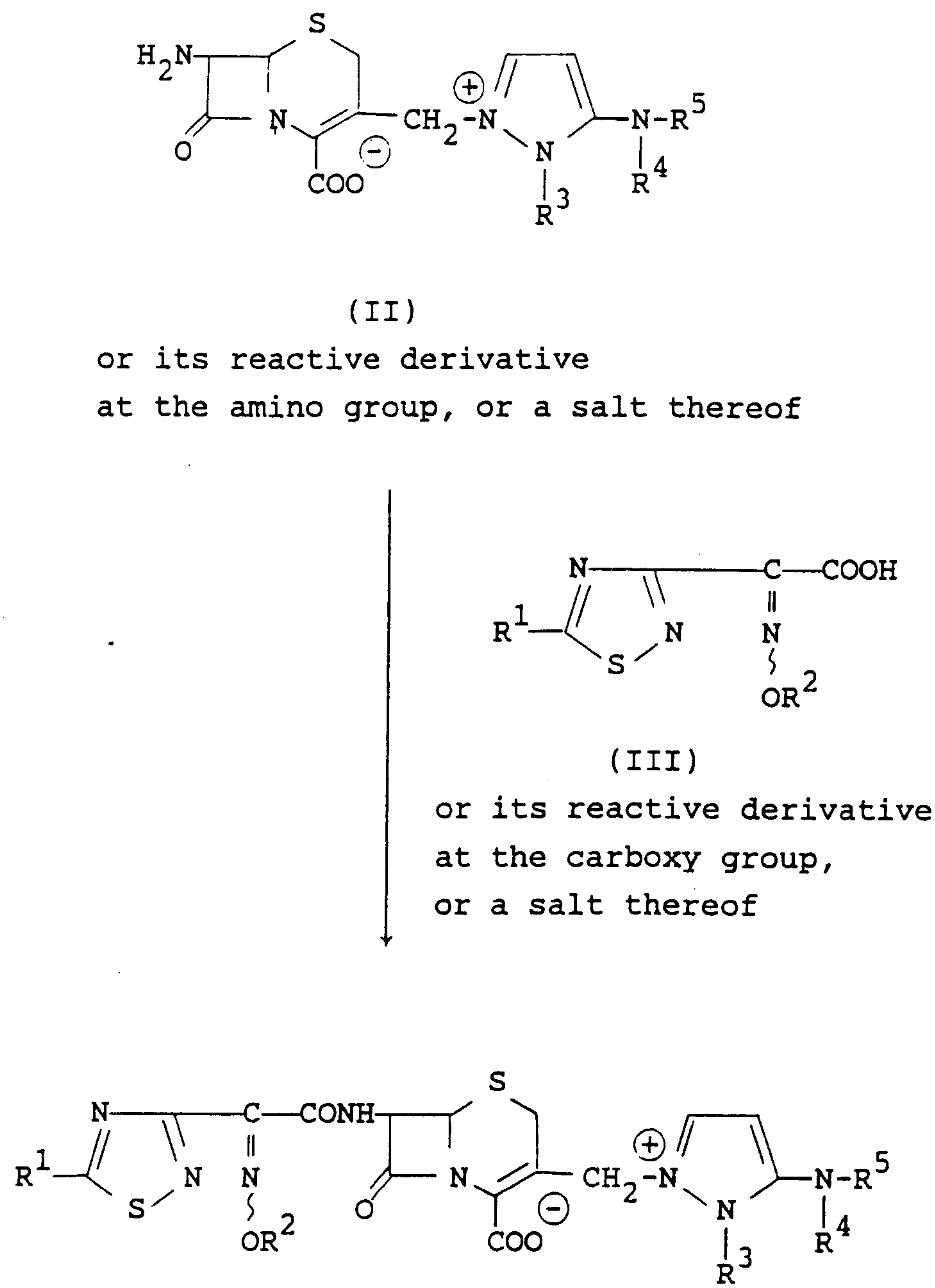

(II)

or its reactive derivative at the amino group, or a salt thereof

(III)

or its reactive derivative at the carboxy group, or a salt thereof

(I)

or a salt thereof

Process (2)

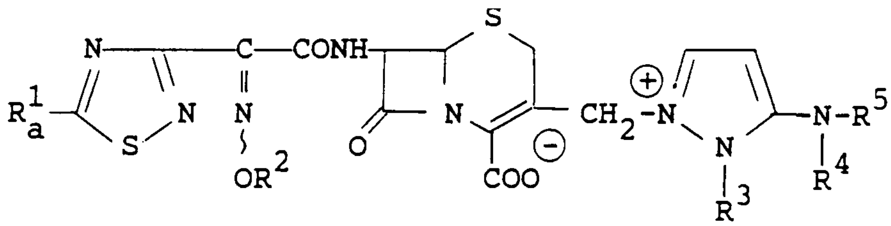

(Ia)
or a salt thereof

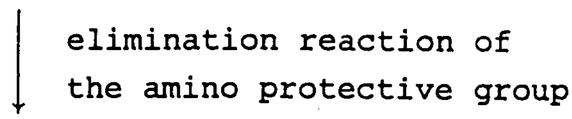


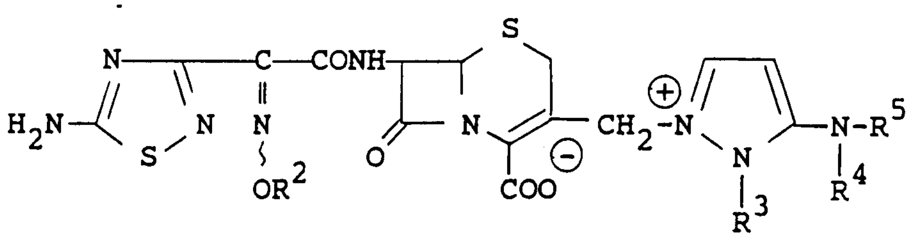

(Ib)
or a salt thereof

wherein

$R^1$, $R^2$, $R^3$, $R^4$ and $R^5$ are each as defined above, and
$R^1_a$ is protected amino.

The starting compound (III) can be prepared by the following processes.

Process (A)

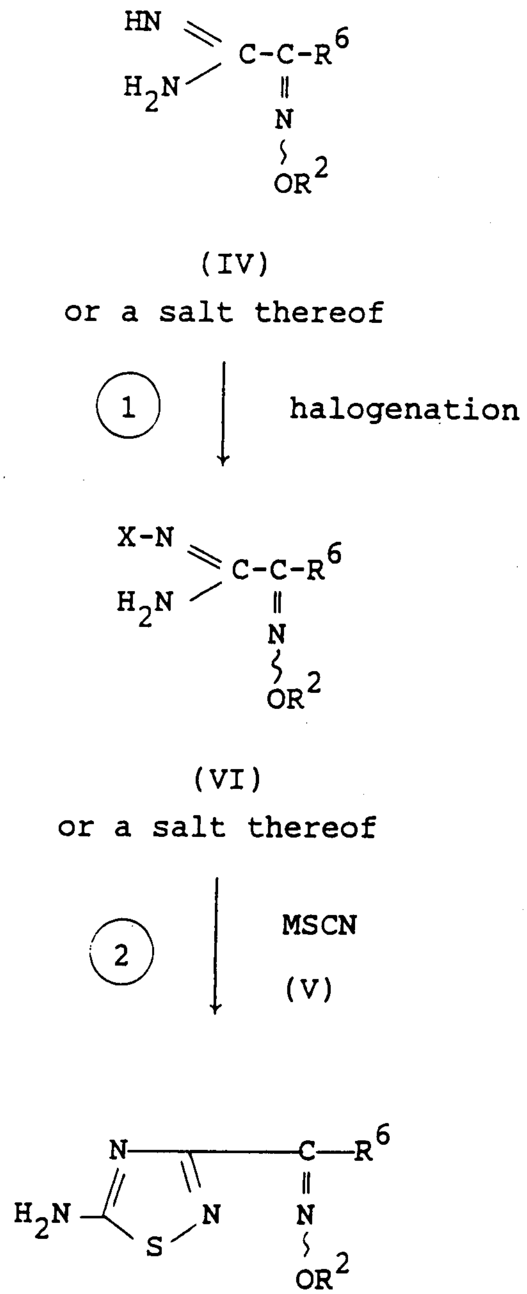


(IV)
or a salt thereof

(VI)
or a salt thereof

(IIIa)
or a salt thereof

Process (B)

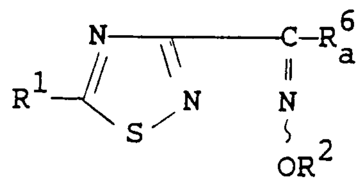

(IIIb)
or a salt thereof

elimination reaction of
the carboxy protective group

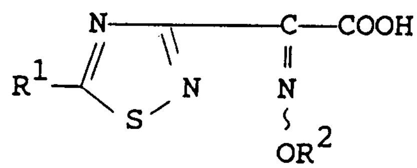

(III)
or a salt thereof

<u>Process (C)</u>

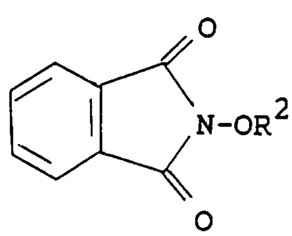

(VII)
or a salt thereof

1

$H_2NNH_2$
(VIII)
or a salt thereof

$H_2N\text{-}OR^2$

(IX)

or a salt thereof

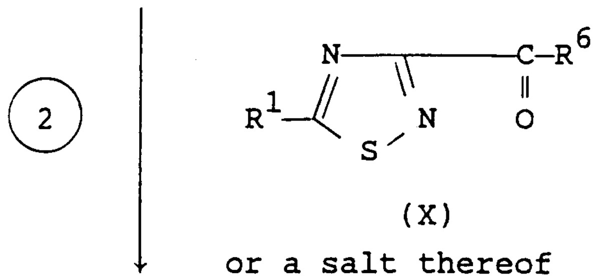


(X)

or a salt thereof

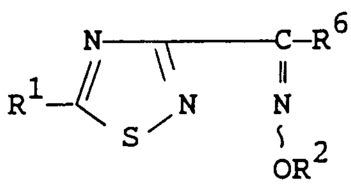

(IIIc)

or a salt thereof

Process (D)

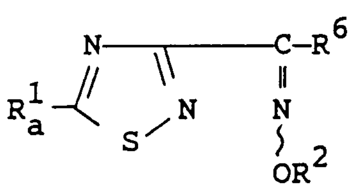

(IIId)

or a salt thereof

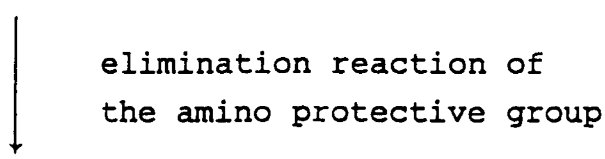

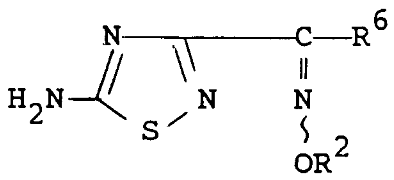

(IIIa)
or a salt thereof

Process (E)

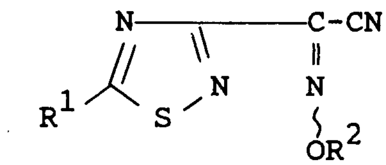

(IIIe)
or a salt thereof

hydrolysis

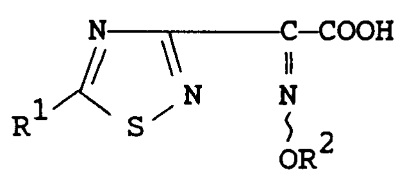

(III)

or a salt thereof

wherein

| | |
|---|---|
| $R^1$, $R^2$ and $R_a^1$ are | each as defined above, |
| $R^6$ is | carboxy, protected carboxy or cyano, |
| $R_a^6$ is | protected carboxy, |
| M is | alkali metal, and |
| X is | halogen. |

The compound (IV) can be prepared in similar manners to those of Preparation 1~5, 19~23 and 27.

The compound (VII) can be prepared in similar manners to those of Preparation 9, 10, 14 and 15.

Regarding the compounds (I), (Ia), (Ib), (III), (IIIa), (IIIb), (IIIc), (IIId) and (IIIe) it is to be understood that said compounds include syn isomer, anti isomer and a mixture thereof.

For example, with regard to the object compound (I), syn isomer means one geometrical isomer having the partial structure represented by the following formula :

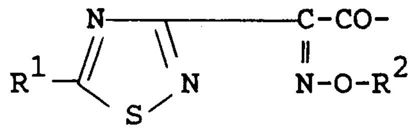

(wherein $R^1$ and $R^2$ are each as defined above) and anti isomer means the other geometrical isomer having the partial structure represented by the following formula :

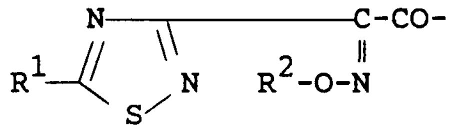

(wherein $R^1$ and $R^2$ are each as defined above), and all of such geometrical isomers and mixture thereof are included within the scope of this invention.

In the present specification and claim, the partial structure of these geometrical isomers and mixture thereof are represented for convenient sake by the following formula :

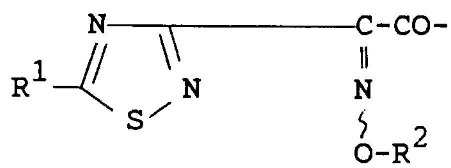

(wherein $R^1$ and $R^2$ are each as defined above).

In the above and subsequent descriptions of the present specification, suitable examples and illustrations of the various definitions which the present invention include within the scope thereof are explained in detail as follows.

The term "lower" is intended to mean 1 to 6 carbon atom(s), preferably 1 to 4 carbon atoms, unless otherwise indicated.

Suitable "lower alkyl moiety" in the terms "lower alkyl substituted with 1 to 6 halogen", "halo(lower)-alkyl", "hydroxy(lower)alkyl" and "protected hydroxy(lower)alkyl" may include straight or branched one such as methyl, ethyl, propyl, isopropyl, butyl, t-butyl, pentyl, hexyl, and the like, in which more preferred one may be $C_1$-$C_4$ alkyl and the most preferred one may be methyl, ethyl or propyl.

Suitable "amino protective group" may include acyl or ar(lower)alkyl which may have suitable substituent(s) (e.g. benzyl, trityl, etc.) or the like.

Suitable "protected amino" may include an acylamino or an amino group substituted by a conventional protecting group such as ar(lower)alkyl which may have suitable substituent(s) (e.g. benzyl, trityl, etc.) or the like.

Suitable "acyl" and "acyl moiety" in the term "acylamino" may include carbamoyl, aliphatic acyl group and acyl group containing an aromatic or heterocyclic ring. And, suitable examples of the said acyl may be lower alkanoyl (e.g. formyl, acetyl, propionyl, butyryl, isobutyryl, valeryl, isovaleryl, oxalyl, succinyl, pivaloyl, etc.); lower alkoxycarbonyl (e.g. methoxycarbonyl, ethoxycarbonyl, propoxycarbonyl, isopropoxycarbonyl, butoxycarbonyl, tertiarybutoxycarbonyl, pentyloxycarbonyl, hexyloxycarbonyl, etc.); lower alkanesulfonyl (e.g. mesyl, ethanesulfonyl, propanesulfonyl, isopropanesulfonyl, butanesulfonyl, etc.); arenesulfonyl (e.g. benzenesulfonyl, tosyl, etc.); aroyl (e.g. benzoyl, toluoyl, xyloyl, naphthoyl, phthaloyl, indancarbonyl, etc.); ar(lower)alkanoyl (e.g. phenylacetyl, phenylpropionyl, etc.); ar(lower)alkoxycarbonyl (e.g. benzyloxycarbonyl, phenethyloxycarbonyl, etc.), and the like. The acyl moiety as stated above may have suitable substituent(s) such as halogen (e.g. chlorine, bromine, iodine or fluorine) or the like.

Suitable "protected carboxy" may include esterified carboxy and the like. And suitable example of said ester may be the ones such as lower alkyl ester (e.g., methyl ester, ethyl ester, propyl ester, isopropyl ester, butyl ester, isobutyl ester, t-butyl ester, pentyl ester, t-pentyl ester, hexyl ester, etc.); lower alkenyl ester (e.g., vinyl ester, allyl ester, etc.); lower alkynyl ester (e.g., ethynyl ester, propynyl ester, etc.); lower alkoxyalkyl ester (e.g., methoxymethyl ester, ethoxymethyl ester, isopropoxymethyl ester, 1-methoxyethyl

ester, 1-ethoxyethyl ester, etc.); lower alkylthioalkyl ester (e.g., methylthiomethyl ester, ethylthiomethyl ester, ethylthioethyl ester, isopropylthiomethyl ester, etc.); mono(or di or tri)halo(lower)alkyl ester (e.g. 2-iodoethyl ester, 2,2,2-trichloroethyl ester, etc.);
lower alkanoyloxy(lower)alkyl ester (e.g., acetoxymethyl ester, propionyloxymethyl ester, butyryloxymethyl ester, valeryloxymethyl ester, pivaloyloxymethyl ester, hexanoyloxymethyl ester, 2-acetoxyethyl ester, 2-propionyloxyethyl ester, etc.); lower alkanesulfonyl(lower)alkyl ester (e.g. mesylmethyl ester, 2-mesylethyl ester etc.); ar(lower)alkyl ester, for example, phenyl(lower)alkyl ester which may have one or more suitable substituent(s) (e.g., benzyl ester, 4-methoxybenzyl ester, 4-nitrobenzyl ester, phenethyl ester, trityl ester, benzhydryl ester, bis(methoxyphenyl)methyl ester, 3,4-dimethoxybenzyl ester, 4-hydroxy-3,5-di-t-butylbenzyl ester, etc.); aryl ester which may have one or more suitable substituent(s) such as substituted or unsubstituted phenyl ester (e.g., phenyl ester, tolyl ester, t-butylphenyl ester, xylyl ester, mesityl ester, cumenyl ester, 4-chlorophenyl ester, 4-methoxyphenyl ester, etc.); tri(lower)alkyl silyl ester; lower alkylthioester (e.g. methylthioester, ethylthioester, etc.) and the like.

Suitable "lower alkylene" may include methylene, ethylene, trimethylene, tetramethylene, pentamethylene, hexamethylene and the like.

Suitable "protective group" in the "protected hydroxy (lower)alkyl" may include acyl as mentioned above, phenyl(lower)alkyl which may have one or more suitable substituent(s) (e.g. benzyl, 4-methoxybenzyl, etc.), tetrahydropyranyl and the like.

Suitable "halogen" and "halogen moiety" in the term "halo(lower)alkyl" may include chlorine, bromine, iodine and fluorine.

Preferable "lower alkyl substituted with 1 to 6 halogen" may be lower alkyl substituted with 1 to 6 fluorine, more preferable one may be ($C_1$-$C_4$)alkyl substituted with 1 to 5 fluorine [e.g. fluoromethyl, difluoromethyl, trifluoromethyl, 1- or 2- fluoroethyl, 1,1-difluoroethyl, 2,2-difluoroethyl, 2,2,2-trifluoroethyl, 1-(or 2- or 3-)fluoropropyl, 1,1-(or 2,2- or 3,3-)difluoropropyl, 3,3,3-trifluoropropyl, 2,2,3,3,3-pentafluoropropyl, etc.].

Suitable "alkali metal" may include sodium, potassium and the like.

Suitable pharmaceutically acceptable salts of the object compound (I) are conventional non-toxic salts and include a metal salt such as an alkali metal salt [e.g. sodium salt, potassium salt, etc.] and an alkaline earth metal salt [e.g. calcium salt, magnesium salt, etc.], an ammonium salt, an organic base salt [e.g. trimethylamine salt, triethylamine salt, pyridine salt, picoline salt, dicyclohexylamine salt, N,N'-dibenzylethylenediamine salt, etc.], an organic acid salt [e.g. formate, acetate, trifluoroacetate, maleate, tartrate, methanesulfonate, benzenesulfonate, toluenesulfonate, etc.], an inorganic acid salt [e.g. hydrochloride, hydrobromide, sulfate, phosphate, etc.], a salt with an amino acid [e.g. arginine salt, aspartic acid salt, glutamic acid salt, etc.], and the like.

Preferred embodiments of the object compound (I) are as follows.

| | |
|---|---|
| $R^1$ is | amino or amino substituted by ar(lower)alkyl which may have suitable substituent(s) [more preferably an amino group substituted by mono(or di or tri)phenyl(lower)alkyl, most preferably tritylamino], |
| $R^2$ is | lower alkyl substituted with 1 to 6 fluorine [more preferably ($C_1$-$C_4$)alkyl substituted with 1 to 5 fluorine, most preferably ($C_1$-$C_3$)alkyl substituted with 1 to 5 fluorine], |
| $R^3$ is | hydroxy(lower)alkyl [more preferably hydroxy($C_1$-$C_4$)alkyl] or fluoro(lower)alkyl [more preferably fluoro($C_1$-$C_4$)alkyl] and |
| $R^4$ is | hydrogen, or |
| $R^3$ and $R^4$ are | linked together to form lower alkylene [more preferably ($C_1$-$C_4$)alkylene], and |
| $R^5$ is | hydrogen. |

The processes for preparing the object and starting compounds of the present invention are explained in detail in the following.

Process (1)

The compound (I) or a salt thereof can be prepared by reacting the compound (II) or its reactive derivative at the amino group, or a salt thereof with the compound (III) or its reactive derivative at the carboxy group, or a salt thereof.

Suitable reactive derivative at the amino group of the compound (II) may include Schiff's base type imino or its tautomeric enamine type isomer formed by the reaction of the compound (II) with a carbonyl compound such as aldehyde, ketone or the like; a silyl derivative formed by the reaction of the compound (II) with a silyl compound such as bis(trimethylsilyl)acetamide, mono(trimethylsilyl)acetamide [e.g. N-(trimethylsilyl)-acetamide], bis(trimethylsilyl)urea or the like; a derivative formed by reaction of the compound (II) with phosphorus trichloride or phosgene, and the like.

Suitable reactive derivative at the carboxy group of the compound (III) may include an acid halide, an acid anhydride, an activated amide, an activated ester, and the like. Suitable examples of the reactive derivatives may be an acid chloride; an acid azide; a mixed acid anhydride with an acid such as substituted phosphoric acid [e.g. dialkylphosphoric acid, phenylphosphoric acid, diphenylphosphoric acid, dibenzylphosphoric acid, halogenated phosphoric acid, etc.], dialkylphosphorous acid, sulfurous acid, thiosulfuric acid, sulfuric acid, sulfonic acid [e.g. methanesulfonic acid, etc.], aliphatic carboxylic acid [e.g. acetic acid, propionic acid, butyric acid, isobutyric acid, pivalic acid, pentanoic acid, isopentanoic acid, 2-ethylbutyric acid, trichloroacetic acid, etc.] or aromatic carboxylic acid [e.g. benzoic acid, etc.]; a symmetrical acid anhydride; an activated amide with imidazole, 4-substituted imidazole, 1-hydroxy-1H-benzotriazole, dimethylpyrazole, triazole or tetrazole; or an activated ester [e.g. cyanomethyl ester, methoxymethyl ester, dimethyliminomethyl $[(CH_3)_2\overset{+}{N}=CH-]$ ester, vinyl ester, propargyl ester, p-nitrophenyl ester, 2,4-dinitrophenyl ester, trichlorophenyl ester, pentachlorophenyl ester, mesylphenyl ester, phenylazophenyl ester, phenyl thioester, p-nitrophenyl thioester, p-cresyl thioester, carboxymethyl thioester, pyranyl ester, pyridyl ester, piperidyl ester, 8-quinolyl thioester, etc.], or an ester with a N-hydroxy compound [e.g. N,N-dimethylhydroxylamine, 1-hydroxy-2-(1H)-pyridone, N-hydroxysuccinimide, N-hydroxyphthalimide, 1-hydroxy-1H-benzotriazole, etc.], and the like. These reactive derivatives can optionally be selected from them according to the kind of the compound (III) to be used.

The reaction is usually carried out in a conventional solvent such as water, alcohol [e.g. methanol, ethanol, etc.], acetone, dioxane, acetonitrile, chloroform, methylene chloride, ethylene chloride, tetrahydrofuran, ethyl acetate, N,N-dimethylformamide, pyridine or any other organic solvent which does not adversely influence the reaction. These conventional solvent may also be used in a mixture with water.

In this reaction, when the compound (III) is used in a free acid form or its salt form, the reaction is preferably carried out in the presence of a conventional condensing agent such as N,N'-dicyclohexylcarbodiimide; N-cyclohexyl-N'-morpholinoethylcarbodiimide; N-cyclohexyl-N'-(4-diethylaminocyclohexyl)-carbodiimide; N,N'-diethylcarbodiimide, N,N'-diisopropylcarbodiimide; N-ethyl-N'-(3-dimethylaminopropyl)-carbodiimide, N,N'-carbonyl-bis(2-methylimidazole); pentamethyleneketene-N-cyclohexylimine, diphenylketene-N-cyclohexylimine; ethoxyacetylene; 1-alkoxy-1-chloroethylene; trialkyl phosphite; ethyl polyphosphate; isopropyl polyphosphate; phosphorus oxychloride (phosphoryl chloride); phosphorus trichloride; thionyl chloride; oxalyl chloride; lower alkyl haloformate [e.g. ethyl chloroformate, isopropyl chloroformate, etc.]; triphenylphosphine; 2-ethyl-7-hydroxybenzisoxazolium salt; 2-ethyl-5-(m-sulfophenyl)isoxazolium hydroxide intramolecular salt; 1-(p-chlorobenzenesulfonyloxy)-6-chloro-1H-benzotriazole; so-called Vilsmeier reagent prepared by the reaction of N,N-dimethylformamide with thionyl chloride, phosgene, trichloromethyl chloroformate, phosphorus oxychloride, etc.; or the like.

The reaction may also be carried out in the presence of an inorganic or organic base such as an alkali metal bicarbonate, tri(lower)alkylamine, pyridine, N-(lower)alkylmorpholine, N,N-di(lower)alkylbenzylamine, or the like.

The reaction temperature is not critical, and the reaction is usually carried out under cooling to warming.

Process (2)

The compound (Ib) or a salt thereof can be prepared by subjecting the compound (Ia) or a salt thereof to elimination reaction of the amino protective group. Suitable method of this elimination reaction may include conventional one such as hydrolysis, reduction and the like.

(i) For Hydrolysis :

The hydrolysis is preferably carried out in the presence of a base or an acid including Lewis acid.

Suitable base may include an inorganic base and an organic base such as an alkali metal [e.g. sodium, potassium, etc.], an alkaline earth metal [e.g. magnesium, calcium, etc.], the hydroxide or carbonate or bicarbonate thereof, trialkylamine [e.g. trimethylamine, triethylamine, etc.], picoline, 1,5-diazabicyclo[4.3.0]-non-5-ene, 1,4-diazabicyclo[2.2.2]octane, 1,8-diazabicyclo[5.4.0]undec-7-ene, or the like.

Suitable acid may include an organic acid [e.g. formic acid, acetic acid, propionic acid, trichloroacetic acid, trifluoroacetic acid, etc.] and an inorganic acid [e.g. hydrochloric acid, hydrobromic acid, sulfuric acid, hydrogen chloride, hydrogen bromide, etc.]. The elimination using Lewis acid such as trihaloacetic acid [e.g. trichloroacetic acid, trifluoroacetic acid, etc.] or the like is preferably carried out in the presence of cation trapping agents [e.g. anisole, phenol, etc.].

The reaction is usually carried out in a solvent such as water, an alcohol [e.g. methanol, ethanol, etc.], methylene chloride, tetrahydrofuran, a mixture thereof or any other solvent which does not

adversely influence the reaction. A liquid base or acid can be also used as the solvent. The reaction temperature is not critical and the reaction is usually carried out under cooling to warming.

(ii) For reduction :

Reduction is carried out in a conventional manner, including chemical reduction and catalytic reduction.

Suitable reducing agents to be used in chemical reduction are a combination of a metal (e.g. tin, zinc, iron, etc.) or metallic compound (e.g. chromium chloride, chromium acetate, etc.) and an organic or inorganic acid (e.g. formic acid, acetic acid, propionic acid, trifluoroacetic acid, p-toluenesulfonic acid, hydrochloric acid, hydrobromic acid, etc.).

Suitable catalysts to be used in catalytic reduction are conventional ones such as platinum catalysts (e.g. platinum plate, spongy platinum, platinum black, colloidal platinum, platinum oxide, platinum wire, etc.), palladium catalysts (e.g. spongy palladium, palladium black, palladium oxide, palladium on carbon, colloidal palladium, palladium on barium sulfate, palladium on barium carbonate, etc.), nickel catalysts (e.g. reduced nickel, nickel oxide, Raney nickel, etc.), cobalt catalysts (e.g. reduced cobalt, Raney cobalt, etc.), iron catalysts (e.g. reduced iron, Raney iron, etc.) copper catalysts (e.g. reduced copper, Raney copper, Ullman copper, etc.) and the like. The reduction is usually carried out in a conventional solvent which does not adversely influence the reaction such as water, methanol, ethanol, propanol, N,N-dimethylformamide, or a mixture thereof. Additionally, in case that the above-mentioned acids to be used in chemical reduction are in liquid, they can also be used as a solvent. Further, a suitable solvent to be used in catalytic reduction may be the above-mentioned solvent, and other conventional solvent such as diethyl ether, dioxane, tetrahydrofuran, etc., or a mixture thereof.

The reaction temperature of this reduction is not critical and the reaction is usually carried out under cooling to warming.

Process (A) - ①

The compound (VI) or a salt thereof can be prepared by subjecting the compound (IV) or a salt thereof to halogenation reaction.

This reaction can be carried out in a similar manner to that of Preparation 6.

Process (A) - ②

The compound (IIIa) or a salt thereof can be prepared by reacting the compound (VI) or a salt thereof with the compound (V).

The reaction is usually carried out in a conventional solvent such as water, alcohol (e.g., methanol, ethanol, isopropyl alcohol, etc.), tetrahydrofuran, dioxane, chloroform, methylene chloride, N,N-dimethyl acetamide, N,N-dimethylformamide or any other organic solvent which does not adversely influence the reaction. Among these solvents, hydrophilic solvents may be used in a mixture with water.

The reaction temperature is not critical and the reaction is usually carried out under cooling to warming.

Process (B)

The compound (III) or a salt thereof can be prepared by subjecting the compound (IIIb) or a salt thereof to elimination reaction of the carboxy protective group.

This reaction can be carried out in a similar manner to that of the aforementioned Process (2), and therefore the reagents to be used and the reaction conditions (e.g. solvent, reaction temperature, etc.) can be referred to those of the Process (2).

Process (C) - ①

The compound (IX) or a salt thereof can be prepared by reacting the compound (VII) or a salt thereof with the compound (VIII) or a salt thereof.

The reaction is usually carried out in a conventional solvent such as water, alcohol (e.g., methanol, ethanol, isopropyl alcohol, etc.), tetrahydrofuran, dioxane, chloroform, methylene chloride, N,N-dimethyl acetamide, N,N-dimethylformamide or any other organic solvent which does not adversely influence the reaction. Among these solvents, hydrophilic solvents may be used in a mixture with water.

The reaction temperature is not critical and the reaction is usually carried out under cooling to warming.

Process (C) - ②

The compound (IIIc) or a salt thereof can be prepared by reacting the compound (IX) or a salt thereof with the compound (X) or a salt thereof.

The reaction is usually carried out in a conventional solvent such as water, alcohol (e.g., methanol, ethanol, isopropyl alcohol, etc.), tetrahydrofuran, dioxane, chloroform, methylene chloride, N,N-dimethyl acetamide, N,N-dimethylformamide or any other organic solvent which does not adversely influence the reaction. Among these solvents, hydrophilic solvents may be used in a mixture with water.

The reaction temperature is not critical and the reaction is usually carried out under cooling to warming.

Process (D)

The compound (IIIa) or a salt thereof can be prepared by subjecting the compound (IIId) or a salt thereof to elimination reaction of the amino protective group. This elimination reaction can be carried out in a similar manner to that of the aforementioned Process (2), and therefore the reagents to be used and the reaction conditions (e.g., solvent, reaction temperature, etc.) can be referred to those of the Process (2).

Process (E)

The compound (III) or a salt thereof can be prepared by subjecting the compound (IIIe) or a salt thereof to hydrolysis reaction.

This hydrolysis can be carried out in a similar manner to that of the aforementioned Process (2), and therefore the reagents to be used and the reaction conditions (e.g., solvent, reaction temperature, etc.) can be referred to those of the Process (2).

Suitable salts of the object and starting compounds and their reactive derivatives in Processes (1), (2) and (A) ~ (E) can be referred to the ones as exemplified for the compound (I).

The object compound (I) and pharmaceutically acceptable salts thereof are novel and exhibit high antimicrobial activity, inhibiting the growth of a wide variety of pathogenic microorganisms including Gram-positive and Gram-negative microorganisms and are useful as antimicrobial agents.

Now in order to show the utility of the object compound (I), the test data on MIC (minimal inhibitory concentration) of representative compound of this invention are shown in the following.

Test method :

In vitro antibacterial activity was determined by the two-fold agar-plate dilution method as described below.

One loopful of an overnight culture of each test strain in Trypticase-soy broth ($10^8$ viable cells per ml) was streaked on heart infusion agar (HI-agar)containing graded concentrations of representative test compound, and the minimal inhibitory concentration (MIC) was expressed in terms of μg/ml after incubation at 37°C for 20 hours.

Test compound :

(1) 7β-[2-(5-Amino-1,2,4-thiadiazol-3-yl)-2-(2-(fluoroethoxyimino)acetamido]-3-(4,5,6,7-tetrahydro-1-pyrazolo[1,5-a]pyrimidinio)methyl-3-cephem-4-carboxylate (syn isomer)

Test result :

| MIC (μg/ml) | |
|---|---|
| Test strain | Test compound (1) |
| E. coli 31 | ≦ 0.025 |

For therapeutic administration, the object compound (I) and pharmaceutically acceptable salts thereof of the present invention are used in the form of conventional pharmaceutical preparation which contains said compound as an active ingredient, in admixture with pharmaceutically acceptable carriers such as an

organic or inorganic solid or liquid excipient which is suitable for oral, parenteral and external administration.

The pharmaceutical preparations may be in solid form such as tablet, granule, powder, capsule, or liquid form such as solution, suspension, syrup, emulsion, lemonade and the like.

In needed, there may be included in the above preparations, auxiliary substances, stabilizing agents, wetting agents and other commonly used additives such as lactose, citric acid, tartaric acid, stearic acid, magnesium stearate, terra alba, sucrose, corn starch, talc, gelatin, agar, pectin, peanut oil, olive oil, cacao butter, ethylene glycol, and the like.

While the dosage of the compound (I) may vary from and also depend upon the age, conditions of the patient, a kind of diseases, a kind of the compound (I) to be applied, etc. In general, amounts between 1 mg and about 4,000 mg or even more per day may be administered to a patient. An average single dose of about 50 mg, 100 mg, 250 mg, 500 mg, 1000 mg of the object compound (I) of the present invention may be used in treating diseases infected by pathogenic microorganisms.

The following Preparations and Examples are given for the purpose of illustrating the present invention in more detail.

Preparation 1

To a solution of dimethyl hydroxyiminomalonate (258 mg) in N,N-dimethylformamide (3 ml) were added 1-bromo-2-fluoroethane (203 mg) and triethylamine (162 mg). After stirred at 60° C for 2.5 hours, the solution was poured into a mixture of ethyl acetate (10 ml) and water (10 ml). The organic layer was washed with a saturated aqueous sodium chloride solution, dried over magnesium sulfate and evaporated in vacuo. The residue was subjected to column chromatography on silica gel (eluent, n-hexane/ethyl acetate; 3/1) to give dimethyl 2-fluoroethoxyiminomalonate (150 mg).

IR (Neat) : 2960, 1760, 1700, 1610, 1440 $cm^{-1}$

NMR ($CDCl_3$, $\delta$) : 3.90 (3H, s), 3.91 (3H, s), 4.45-4.50 (1H, m), 4.50-4.70 (2H, m), 4.75-4.85 (1H, m)

Preparation 2

To a solution of dimethyl 2-fluoroethoxyiminomalonate (122 mg) in methanol (0.5 ml) was added 28% ammonia water (140 ml) under ice-cooling. After stirred at the same temperature for 30 minutes, the solution was poured into a mixture of ethyl acetate and water, and adjusted to pH 7.0 with conc. hydrochloric acid. The organic layer was washed with a saturated aqueous sodium chloride solution, dried over magnesium sulfate and evaporated in vacuo to give (Z)-methyl 2-carbamoyl-2-(2-fluoroethoxyimino)-acetate (94 mg).

IR (Nujol) : 3450, 3200, 1740, 1690 $cm^{-1}$

NMR ($CDCl_3$, $\delta$) : 3.92 (3H, s), 4.40-4.45 (1H, m), 4.50-4.60 (2H, m), 4.75-4.80 (1H, m), 5.93 (1H, br s), 6.45 (1H, br s)

Preparation 3

To a solution of (Z)-methyl 2-carbamoyl-2-(2-fluoroethoxyimino)acetate (60 mg) in pyridine (600 ml) was added trifluoroacetic anhydride (120 ml) under ice-cooling. After stirred at room temperature for 40 minutes, the solution was poured into a mixture of ethyl acetate and water. The organic layer was washed with 1N-hydrochloric acid and a saturated aqueous sodium chloride solution, dried over magnesium sulfate and evaporated in vacuo to give (Z)-methyl 2-cyano-2-(2-fluoroethoxyimino)acetate (88 mg).

IR (Neat) : 2980, 2260, 1750, 1560, 1450 $cm^{-1}$

NMR ($CDCl_3$, $\delta$) : 3.92 (3H, s), 4.59 (2H, s), 4.70-4.75 (1H, m), 4.80-4.85 (1H, m)

Preparation 4

To a solution of (Z)-methyl 2-cyano-2-(2-fluoroethoxyimino)acetate (1.74 g) in methanol (44 ml) was added 28% sodium methoxide in methanol (965 ml). After stirred at room temperature for 30 minutes, methanol was evaporated and the residue was poured into a mixture of ethyl acetate and water. The organic layer was dried over magnesium sulfate and evaporated in vacuo. The residue was subjected to column chromatography on silica gel (eluent, n-hexane/ethyl acetate; 3/1) to give (Z)-methyl 3-imino-3-methoxy-2-(2-fluoroethoxyimino)propionate (0.80 g).

IR (Neat) : 3320, 2960, 1740, 1660 $cm^{-1}$

NMR ($CDCl_3$, $\delta$) : 3.90 (3H, s), 3.91 (3H, s), 4.35-4.40 (1H, m), 4.45-4.55 (2H, m), 4.75-4.80 (1H, m), 8.26

(1H, br s)

Preparation 5

To a solution of (Z)-methyl 3-imino-3-methoxy-2-(2-fluoroethoxyimino)propionate (790 mg) in methanol (17.4 ml) was added ammonium chloride (615 mg). After stirred under reflux for 5 hours, the mixture was poured into ethyl acetate. After removal of the precipitate by filtration, the filtrate was evaporated in vacuo and triturated with diethyl ether to give (Z)-methyl 2-amidino-2-(2-fluoroethoxyimino)acetate hydrochloride (660 mg).
IR (Nujol): 3100, 1750, 1680, 1605 $cm^{-1}$
NMR (DMSO-$d_6$, $\delta$) : 3.87 (3H, s), 4.50-4.55 (1H, m), 4.60-4.70 (2H, m), 4.85-4.90 (1H, m), 9.66 (3H, br s)

Preparation 6

To a solution of (Z)-methyl 2-amidino-2-(2-fluoroethoxyimino)acetate hydrochloride (640 mg) in methanol (8.0 ml) were added triethylamine (654 mg) and bromine (449 mg) at -15°C. After stirred at the same temperature for 15 minutes, a solution of potassium thiocyanate (273 mg) in methanol (3.0 ml) was added to the mixture at -15°C with stirring. After stirred at -5°C for 2 hours, methanol was evaporated and the residue was poured into a mixture of ethyl acetate and water. The organic layer was washed with a saturated aqueous sodium chloride solution, dried over magnesium sulfate and evaporated in vacuo. The residue was subjected to column chromatography on silica gel (eluent, n-hexane/ethyl acetate; 1/1) to give methyl 2-(5-amino-1,2,4-thiadiazol-3-yl)-2-(2-fluroethoxyimino)acetate (syn isomer) (320 mg).
IR (Nujol) : 3400, 3250, 3100, 1740, 1620 $cm^{-1}$
NMR (DMSO-$d_6$, $\delta$) : 3.83 (3H, s), 4.35-4.40 (1H, m), 4.50-4.55 (2H, m), 4.75-4.80 (1H, m), 8.26 (2H, s)

Preparation 7

To a solution of methyl 2-(5-amino-1,2,4-thiadiazol-3-yl)-2-(2-fluoroethoxyimino)acetate (syn isomer) (12.1 g) in methanol (350 ml) was added 1N-sodium hydroxide solution (195 ml). After stirred at 50°C for 30 minutes, methanol was evaporated. The residue was poured into a mixture of ethyl acetate, tetrahydrofuran and water, and adjusted to pH 2.0 with 1N-hydrochloric acid. The organic layer was dried over magnesium sulfate, evaporated in vacuo and triturated with diisopropyl ether to give 2-(5-amino-1,2,4-thiadiazol-3-yl)-2-(2-fluoroethoxyimino)-acetic acid (syn isomer) (9.31 g)
IR (Nujol): 3400, 3150, 1700, 1620, 1540 $cm^{-1}$
NMR (DMSO-$d_6$, $\delta$) : 4.30-4.35 (1H, m), 4.45-4.55 (2H, m), 4.75-4.80 (1H, m), 8.22 (1H, s)

Preparation 8

A solution of phosphorus pentachloride (2.80 g) in dichloromethane (30 ml) was stirred at room temperature for 20 minutes. 2-(5-Amino-1,2,4-thiadiazol-3-yl)-2-(2-fluoroethoxyimino)acetic acid (syn isomer) (3.0 g) was added to the solution at -5°C with stirring. After stirred at the same temperature for 1.5 hours, diisopropyl ether (90 ml) was added to the mixture and triturated to give 2-(5-amino-1,2,4-thiadiazol-3-yl)-2-(2-fluoroethoxyimino)acetyl chloride hydrochloride (syn isomer) (2.22 g).
IR (Nujol): 3250, 1780, 1635 $cm^{-1}$
NMR (DMSO-$d_6$, $\delta$) : 4.30-4.35 (1H, m), 4.45-4.55 (2H, m), 4.75-4.80 (1H, m), 11.4 (2H, br s)

Preparation 9

To a mixture of p-toluenesulfonyl chloride (25.56 g) and water (42.7 ml) were added 2,2-difluoroethanol (10.0 g) and a solution of sodium hydroxide (5.85 g) in water (23.2 ml). After stirred at 50°C for 1.5 hours, the solution was poured into ethyl acetate (100 ml). The separated organic layer was washed with a saturated aqueous sodium chloride solution, dried over magnesium sulfate and evaporated in vacuo to give 2,2-difluoroethyl p-toluenesulfonate (25.84 g).
IR (Nujol): 1595, 1180 $cm^{-1}$
NMR (DMSO-$d_6$, $\delta$) : 2.44 (3H, s), 4.35 (2H, dt, J=2.8Hz, 15.1Hz), 6.25 (1H, tt, J=2.8Hz, 53.3Hz), 7.51 (2H, d, J=8Hz), 7.83 (2H, J=8.0Hz)

Preparation 10

To a solution of N-hydroxyphthalimide (163 mg) and 1,8-diazabicyclo[5.4.0]undec-7-ene (167 mg) in N,N-dimethylformamide (2.0 ml) was added 2,2-difluoroethyl p-toluenesulfonate (260 mg). After stirred at 90°C for 3 hours, the solution was poured into a mixture of water (10 ml) and ethyl acetate (10 ml). The separated organic layer was washed with a saturated aqueous sodium chloride solution twice, dried over magnesium sulfate and evaporated in vacuo. The residue was recrystallized from isopropyl ether to give N-(2,2-difluoroethoxy)phthalimide (141 mg).

IR (Nujol): 1800, 1740, 1720 $cm^{-1}$

NMR ($CDCl_3$, $\delta$) : 4.38 (2H, dt, J=4.2Hz, 12.7Hz), 6.23 (1H, tt, J=4.2Hz, 54.5Hz), 7.7-8.0 (4H, m)

Preparation 11

To a solution of N-(2,2-difluoroethoxy)phthalimide (545 mg) in ethanol (2.4 ml) was added hydrazine hydrate (120 mg). After the mixture was stirred under reflux for 5 minutes, a precipitate was collected by filtration to give O-(2,2-difluoroethyl)hydroxylamine (compound A). On the other hand, to a solution of sodium hydroxide (240 mg) in water (3.0 ml) was added S-methyl 2-(5-formamido-1,2,4-thiadiazol-3-yl)-2-oxo-thioacetate (776 mg). After stirred at room temperature for 1 hour, the solution was adjusted to pH 7.0 with 6N-hydrochloric acid under ice-cooling. To the cold solution was added the ethanol solution of compound A obtained above. After adjusted to pH 3.5 with 6N-hydrochloric acid, the mixture was stirred at room temperature for 3.5 hours. After the precipitate was removed by filtration, the ethanol was evaporated in vacuo, and the resulting solution was washed with ethyl acetate at pH 7.0. The solution was adjusted to pH 0.9 with 6N-hydrochloric acid under ice-cooling, and the resulting precipitate was collected by filtration and washed with cold water to give 2-(5-formamido-1,2,4-thiadiazol-3-yl)-2-(2,2-difluoroethoxyimino)acetic acid (syn isomer) (220 mg).

IR (Nujol) : 3400, 1720, 1685 $cm^{-1}$

NMR (DMSO-$d_6$, $\delta$) : 3.50 (1H, br s), 4.53 (2H, dt, J=3.6Hz, 14.7Hz), 6.29 (1H, tt, J=3.6Hz, 54.3Hz), 8.85 (1H, s), 13.57 (1H, s)

Preparation 12

To a solution of sodium hydroxide (240 mg) in water (3 ml) was added 2-(5-formamido-1,2,4-thiadiazol-3-yl)-2-(2,2-difluoroethoxyimino)acetic acid (syn isomer) (560 mg). After stirred at 50°C for 30 minutes, the solution was cooled to room temperature, adjusted to pH 7.0 with 1N-hydrochloric acid and washed with ethyl acetate. The solution was adjusted to pH 0.9 with 1N-hydrochloric acid and extracted with ethyl acetate. The extract was dried over magnesium sulfate and evaporated in vacuo to give 2-(5-amino-1,2,4-thiadiazol-3-yl)-2-(2,2-difluoroethoxyimino)acetic acid (syn isomer) (370 mg).

IR (Nujol) : 3400, 1720, 1620 $cm^{-1}$

NMR (DMSO-$d_6$, $\delta$) : 4.46 (2H, dt, J=3.6Hz, 14.7Hz), 6.24 (1H, tt, J=3.6Hz, 54.4Hz), 8.24 (2H, s)

Preparation 13

The following compound was obtained according to a similar manner to that of Preparation 8.

2-(5-Amino-1,2,4-thiadiazol-3-yl)-2-(2,2-difluoroethoxyimino)acetyl chloride hydrochloride (syn isomer).

Preparation 14

The following compound was prepared according to a similar manner to that of Preparation 9.

2,2,3,3,3-Pentafluoropropyl p-toluenesulfonate

NMR (DMSO-$d_6$, $\delta$) : 2.44 (3H, s), 4.94 (2H, t, J=13.1Hz), 7.52 (2H, d, J=8.1Hz), 7.88 (2H, d, J=8.1Hz)

Preparation 15

The following compound was prepared according to a similar manner to that of Preparation 10.

N-(2,2,3,3,3-Pentafluoropropoxy)phthalimide

IR (Nujol) : 1780, 1740 $cm^{-1}$

NMR ($CDCl_3$, $\delta$) : 4.66 (2H, t, J=12.9Hz), 7.7-8.0 (4H, m)

Preparation 16

The following compound was prepared according to a similar manner to that of Preparation 11.

2-(5-Formamido-1,2,4-thiadiazol-3-yl)-2-(2,2,3,3,3-pentafluoropropoxyimino)acetic acid (syn isomer)

IR (Nujol) : 3250, 1730, 1690 $cm^{-1}$

NMR ($DMSO-d_6$, $\delta$) : 3.38 (1H, br s), 5.05 (2H, t, J = 13.7Hz), 8.86 (1H, s), 13.53 (1H, s)

Preparation 17

The following compound was prepared according to a similar manner to that of Preparation 12.

2-(5-Amino-1,2,4-thiadiazol-3-yl)-2-(2,2,3,3,3-pentafluoropropoxyimino)acetic acid (syn isomer)

IR (Nujol) : 3300, 1710, 1620 $cm^{-1}$

NMR ($DMSO-d_6$, $\delta$) : 3.60 (1H, br s), 4.98 (2H, t, J = 13.4Hz), 8.27 (2H, s)

Preparation 18

The following compound was prepared according to a similar manner to that of Preparation 8.

2-(5-Amino-1,2,4-thiadiazol-3-yl)-2-(2,2,3,3,3-pentafluoropropoxyimino)acetyl chloride hydrochloride (syn isomer)

IR (Nujol) : 1750, 1630 $cm^{-1}$

NMR ($DMSO-d_6$, $\delta$) : 4.98 (2H, t, J = 13.6Hz), 14.12 (2H, br s)

Preparation 19

To a solution of dimethyl hydroxyiminomalonate (1.61 g) in N,N-dimethylformamide (16 ml) were added 2-bromo-1,1-difluoroethane (1.74 g) and potassium carbonate (1.66 g). After stirred at 50 °C for 3 hours, the mixture was poured into a mixture of water and ethyl acetate. The solution was adjusted to pH 8.0 with 6N-hydrochloric acid and extracted with ethyl acetate. The extract was washed with a saturated sodium chloride solution three times, dried over magnesium sulfate, and concentrated under reduced pressure to give dimethyl 2,2-difluoroethoxyiminomalonate (1.39 g).

IR (Film) : 1740, 1610, 1440, 1350, 1270 $cm^{-1}$

NMR ($CDCl_3$, $\delta$) : 3.91 (3H, s), 3.92 (3H, s), 4.46 (2H, dt, J = 4.2Hz, J = 13.0Hz), 6.00 (1H, tt, J = 4.2Hz, J = 54.8Hz)

Preparation 20

To a solution of dimethyl 2,2-difluoroethoxyiminomalonate (1.37 g) in methanol (5.6 ml) was added 28% ammonia aqueous solution (1.45 ml) at ice-cooling. After stirred at ice-cooling for 1 hour, the solution was adjusted to pH 7.0 with 6N-hydrochloric acid and methanol was evaporated under reduced pressure. The residue was poured into ethyl acetate and extracted with ethyl acetate. The extract was washed with a saturated sodium chloride solution, dried over magnesium sulfate, and concentrated under reduced pressure to give (Z)-methyl 2-carbamoyl-2-(2,2-difluoroethoxyimino)acetate (1.11 g).

IR (Nujol) : 3350, 3230, 1750, 1680, 1300 $cm^{-1}$

NMR ($CDCl_3$, $\delta$) : 3.92 (3H, s), 4.41 (2H, dt, J = 4.2Hz, J = 13.0Hz), 5.98 (1H, tt, J = 4.2Hz, J = 54.7Hz), 5.97 (1H, br s), 6.42 (1H, br s)

Preparation 21

To a solution of (Z)-methyl-2-carbamoyl-2-(2,2-difluoroethoxyimino)acetate (1.09 g) in pyridine (10.9 ml) was added trifluoroacetic anhydride (2.18 ml) at ice-cooling. After stirred at room temperature for 40 minutes, the solution was poured into a mixture of ice water and ethyl acetate and extracted with ethyl acetate. The extract was washed with 1N-hydrochloric acid, aqueous sodium hydrogencarbonate solution and a saturated sodium chloride solution three times successively, dried over magnesium sulfate and concentrated under reduced pressure to give (Z)-methyl 2-cyano-2-(2,2-difluoroethoxyimino)-acetate (0.97 g).

IR (Film) : 2220, 1750, 1560, 1440, 1260 $cm^{-1}$

NMR ($CDCl_3$, $\delta$) : 3.94 (3H, s), 4.57 (2H, dt, J = 4.2Hz, J = 12.9Hz), 6.03 (1H, tt, J = 4.2Hz, J = 54.5Hz)

Preparation 22

To a solution of N,N-dimethylformamide (219 mg) in dichloromethane (2.1 ml) was added phosphorus oxychloride (399 mg) at 10° C. After stirred at 10° C for 30 minutes, the solution was cooled at -10° C. To the cold solution were added (Z)-methyl-2-carbamoyl-2-(2,2-difluoroethoxyimino)acetate (420 mg) and pyridine (791 mg) successively at -10 ~ -5° C. After stirred at -10 ~ -5° C for 1 hour, the mixture was poured into water, and extracted with dichloromethane twice. The dichloromethane solution was washed with water, dried over magnesium sulfate and concentrated under reduced pressure to give (Z)-methyl-2-cyano-2-(2,2-difluoroethoxyimino)acetate (381 mg).

IR (Film) : 2220, 1750, 1560, 1440, 1260 $cm^{-1}$

NMR ($CDCl_3$, $\delta$) : 3.94 (3H, s), 4.57 (2H, dt, J=4.2Hz, J=12.9Hz), 6.03 (1H, tt, J=4.2Hz, J=54.5Hz)

Preparation 23

To a solution of (Z)-methyl 2-cyano-2-(2,2-difluoroethoxyimino)acetate (1.0 g) in methanol (20 ml) was added sodium methylate (28% in methanol) (521 µl) at ice-cooling. After stirred at ice-cooling for 30 minutes, the solution was adjusted to pH 7.0 with 6N-hydrochloric acid and methanol was evaporated under reduced pressure. The residue was poured into ethyl acetate and extracted with ethyl acetate. The extract was washed with a saturated sodium chloride solution, dried over magnesium sulfate, and concentrated under reduced pressure to give (Z)-methyl 2-(2,2-difluoroethoxyimino)-3-imino-3-methoxypropionate (0.64 g).

IR (Film) : 3300, 2950, 1740, 1650, 1440, 1290 $cm^{-1}$

NMR ($CDCl_3$, $\delta$) : 3.88 (3H, s), 3.91 (3H, s), 4.40 (2H, dt, J=4.2Hz, J=13.0Hz), 5.98 (1H, tt, J=4.2Hz, J=54.9Hz), 8.25 (1H, br s)

Preparation 24

To a solution of (Z)-methyl-2-(2,2-difluoroethoxyimino)-3-imino-3-methoxypropionate (448 mg) in methanol (6.7 ml) was added ammonium chloride (321 mg). After stirred at 60° C for 2 hours, the mixture was poured into ethyl acetate (50 ml). After removal of insoluble material by filtration, the filtrate was concentrated under reduced pressure. Diisopropyl ether (13 ml) was poured into the residue. The resulting crystals were collected by filtration, washed with diisopropyl ether and dried to give (Z)-methyl 2-amidino-2-(2-difluoroethoxyimino)acetate hydrochloride (0.32 g).

IR (Nujol) : 3250, 2920, 1750, 1680 $cm^{-1}$

NMR (DMSO-$d_6$, $\delta$) : 3.87 (3H, s), 4.66 (2H, dt, J=3.5Hz, J=14.5Hz), 6.45 (1H, tt, J=3.5Hz, J=54.1Hz), 9.73 (3H, br s)

Preparation 25

To a solution of (Z)-methyl 2-amidino-2-(2,2-difluoroethoxyimino)acetate hydrochloride (2.1 g) in methanol (21 ml) were added triethylamine (1.99 g) and bromine (1.37 g) at -13° C. After the solution was stirred at -13 ~ -10° C for 15 minutes, a solution of potassium thiocyanate (831 mg) in methanol (8.3 ml) was added to the solution at -10° C. After stirred at room temperature overnight, the solution was poured into a mixture of ethyl acetate and water, and extracted with ethyl acetate. The extract was washed with a saturated sodium chloride solution, dried over magnesium sulfate, and concentrated under reduced pressure. The residue was subjected to column chromatography on silica gel (40 g; eluent n-hexane/ethyl acetate 2/1) to give methyl 2-(5-amino-1,2,4-thiadiazol-3-yl)-2-(2,2-difluoroethoxyimino)acetate (syn isomer) (0.64 g).

IR (Nujol) : 3100, 1740, 1630, 1540 $cm^{-1}$

NMR (DMSO-$d_6$, $\delta$) : 3.84 (3H, s), 4.50 (2H, dt, J=3.5Hz, J=14.7Hz), 6.25 (1H, tt, J=3.5Hz, J=54.3Hz), 8.29 (2H, br s)

Preparation 26

To a solution of methyl 2-(5-amino-1,2,4-thiadiazol3-yl)-2-(2,2-difluoroethoxyimino)acetate (syn isomer) (266 mg) in water (4 ml) was added 1N-sodium hydroxide solution (1 ml). After stirred at room temperature for 2 hours, the solution was adjusted to pH 3.6 with 6N-hydrochloric acid and washed with ethyl acetate. The aqueous solution was adjusted to pH 1.0 with 6N-hydrochloric acid and extracted with ethyl acetate. The extract was dried over magnesium sulfate and concentrated under reduced pressure to give 2-(5-amino-1,2,4-thiadiazol-3-yl)-2-(2,2-difluoroethoxyimino)acetic acid (syn isomer) (0.21 g).

IR (Nujol) : 3300, 1710, 1620 $cm^{-1}$
NMR ($DMSO-d_6$, δ) : 4.46 (2H, dt, J=3.6Hz, J=14.7Hz), 6.24 (1H, tt, J=3.6Hz, J=54.4Hz), 8.24 (2H, s)

Preparation 27

To a solution of sodium oxidoiminopropanedinitrile (1.17 g) in N,N-dimethylformamide (12 ml) was added 2-bromo-1,1-difluoroethane (1.74 g). After stirred at 85°C for 9 hours, the mixture was poured into a mixture of water and diisopropyl ether and extracted with diisopropyl ether. The extract was washed with a saturated sodium chloride solution three times, dried with magnesium sulfate, and concentrated under reduced pressure to give 2,2-difluoroethoxyiminopropanedinitrile (378 mg).
IR (Film) : 2250, 1530, 1250 $cm^{-1}$
NMR ($CDCl_3$, δ) : 4.72 (2H, dt, J=3.9Hz, J=12.8Hz), 6.06 (1H, tt, J=3.9Hz, J=54.0Hz)

Preparation 28

To a mixture of ammonium chloride (321 mg), 28% ammonia aqueous solution (1.34 ml) and N,N-dimethylformamide (0.6 ml) was added a solution of 2,2-difluoroethoxyiminopropanedinitrile (318 mg) in N,N-dimethylformamide (0.4 ml) at -7°C. After stirred at -5 ~ 7°C for 30 minutes, the solution was poured into a mixture of water and dichloromethane and extracted with dichloromethane three times. The extract was dried with magnesium sulfate and evaporated under reduced pressure. To the residue were added diisopropyl ether (5 ml) and acetic acid (180 mg). The resulting crystals were collected by filtration, washed with diisopropyl ether and dried to give (Z)-2-amidino-2-(2,2-difluoroethoxyimino)-ethanenitrile acetate (342 mg).
IR (Nujol) : 3150, 1660, 1570 $cm^{-1}$
NMR ($DMSO-d_6$, δ) : 1.91 (3H, s), 4.69 (2H, dt, J=3.5Hz, J=14.6Hz), 6.43 (1H, tt, J=3.5Hz, J=54.1Hz), 7.38 (3H, br s)

Preparation 29

To a solution of (Z)-2-amidino-2-(2,2-difluoroethoxyimino)ethanenitrile acetate (1.57 g) in methanol (15.7 ml) were added triethylamine (1.55 g) and bromine (1.06 g) at -13°C. After the solution was stirred at -13 ~ -10°C for 15 minutes, a solution of potassium thiocyanate (646 mg) in methanol (6.5 ml) was added to the solution at -10°C. After the solution was stirred at -5 ~ -10°C for 1 hour, the resulting crystals were collected by filtration washed with cold water to give 2-(5-amino-1,2,4-thiadiazol-3-yl)-2-(2,2-difluoroethoxyimino)ethanenitrile (syn isomer) (975 mg).
IR (Nujol) : 3450, 3300, 3150, 1640, 1550 $cm^{-1}$
NMR ($DMSO-d_6$, δ) : 4.76 (2H, dt, J=3.2Hz, J=14.9Hz), 6.38 (1H, tt, J=3.3Hz, J=53.8Hz), 8.44 (2H, s)

Preparation 30

To a mixture of 2-(5-amino-1,2,4-thiadiazol-3-yl)-2-(2,2-difluoroethoxyimino)ethanenitrile (syn isomer) (233 mg) and water (1 ml) was added sodium hydroxide (200 mg). After stirred at 60°C for 5 hours, the solution was adjusted to pH 4.0 with 6N-hydrochloric acid and washed with ethyl acetate. The aqueous solution was adjusted to pH 1.0 with 6N-hydrochloric acid and extracted with ethyl acetate twice. The extract was dried over magnesium sulfate and concentrated under reduced pressure to give 2-(5-amino-1,2,4-thiadiazol-3-yl)-2-(2,2-difluoroethoxyimino)acetic acid (syn isomer) (192 mg).
NMR ($DMSO-d_6$, δ) : 4.46 (2H, dt, J=3.6Hz, J=14.7Hz), 6.24 (1H, tt, J=3.6Hz, J=54.4Hz), 8.24 (2H, s)

Preparation 31

To a solution of 2-(5-amino-1,2,4-thiadiazol-3-yl)-2-(2,2-difluoroethoxyimino)acetic acid (syn isomer) (10 g) in N,N-dimethylacetamide (100 ml) were added dropwise methanesulfonyl chloride (6.14 ml) and potassium carbonate (5.48 g) under stirring at 5°C, and then the stirring was continued for 1.5 hours at the same temperature. The reaction mixture was poured into a mixture of ethyl acetate (1000 ml), 6N-hydrochloric acid (4 ml) and cold water (1.4 ℓ). The organic layer was separated, washed with water and brine, dried over magnesium sulfate and then concentrated under reduced pressure to give crystals, which were collected by filtration and dried to give methanesulfonic 2-(5-amino-1,2,4-thiadiazol-3-yl)-2-(2,2-difluoroethoxyimino)acetic anhydride (syn isomer) (9.2 g).

mp : 125-128 ° C
IR (Nujol) : 3430, 3230, 1785, 1605, 1530 $cm^{-1}$
NMR ($CD_3COCD_3$, δ ) : 3.68 (3H, s), 4.62 (2H, dt, J = 3.7Hz, J = 14.1Hz), 6.27 (1H, tt, J = 3.7Hz, J = 54.6Hs), 7.84 (2H, br s)

Preparation 32

To a mixture of dimethylmalonate (306 g) and water (918 ml) were added sodium nitrite (192 g) and acetic acid (279 g). After stirred at room temperature for 1 hour, the mixture was stirred at 35 ° C for 20 hours. The solution was poured into a dichloroethane and extracted with dichloromthane twice. The extract was dried over magnesium sulfate and concentrated under reduced pressure. Toluene (600 ml) was added to the residue and the solution was concentrated under reduced pressure to give dimethyl hydroxyiminomalonate (317.7 g).
NMR ($CDCl_3$, δ) : 3.90 (3H, s), 3.94 (3H, s), 9.60 (1H, br s)

Example 1

(1) To a solution of 7β-amino-3-[2,3-dihydro-2-(1H-imidazo[1,2-b]pyrazolio)]methyl-3-cephem-4-carboxylate hydrochloride (1 g) and N-(trimethylsilyl)acetamide (5 g) in tetrahydrofuran (10 ml) and N,N-dimethylformamide (10 ml) was added 2-(5-amino-1,2,4-thiadiazol-3-yl)-2-fluoromethoxyiminoacetyl chloride hydrochloride (syn isomer) under ice-cooling. After being stirred at the same temperature, the mixture was poured into ethyl acetate. The resulting precipitate was collected by filtration, dissolved in water, and adjusted to pH 3 with a sodium bicarbonate aqueous solution. The solution was subjected to column chromatography on Diaion HP-20 (Trademark : Mitsubishi Kasei Corporation), and eluted with 10% isopropyl alcohol. The fractions containing the object compound was combined and lyophilized to give 7β-[2-(5-amino-1,2,4-thiadiazol-3-yl)-2-fluoromethoxyiminoacetamido]-3-[2,3-dihydro-5-(1H-imidazo-[1,2-b]pyrazolio)]methyl-3-cephem-4-carboxylate (syn isomer) (651 mg).
IR (Nujol) : 1760, 1650, 1595 $cm^{-1}$
NMR (DMSO-$d_6$, δ) : 3.12 and 3.34 (2H, ABq, J = 17.3Hz), 4.01 (2H, m), 4.24 (1H, m), 4.50 (1H, m), 5.03 (2H, m), 5.04 (1H, d, J = 4.6Hz), 5.65 (1H, dd, J = 4.6Hz, 8.3Hz), 5.77 (2H, d, J = 54.5Hz), 5.87 (1H, d, J = 3.0Hz), 8.00 (1H, br s), 8.24 (1H, d, J = 3.0Hz), 8.27 (2H, br s), 8.71 (1H, d, J = 8.3Hz)

The following compounds were obtained according to a similar manner to that of Example 1-(1).

(2) 7β-[2-(5-Amino-1,2,4-thiadiazol-3-yl)-2-fluoromethoxyiminoacetamido]-3-[5-amino-1-(2-hydroxyethyl)-2-pyrazolio]methyl-3-cephem-4-carboxylate (syn isomer)
IR (Nujol) : 3200, 1760, 1650, 1600 $cm^{-1}$
NMR (DMSO-$d_6$, δ) : 2.91 and 3.21 (2H, d, J = 17.0Hz), 3.59 (2H, m), 4.35 (2H, m), 5.03 (1H, d, J = 4.9Hz), 5.13 (2H, m), 5.64 (1H, d, J = 8.3Hz), 5.77 (2H, d, J = 52.0Hz), 5.81 (1H, d, J = 3.2Hz), 7.30 (2H, br s), 8.10 (1H, d, J = 3.2Hz), 8.23 (2H, br s), 9.73 (1H, d, J = 8.3Hz)

(3) 7β-[2-(5-Amino-1,2,4-thiadiazol-3-yl)-2-(2-fluoroethoxyimino)acetamido]-3-[5-amino-1-(2-hydroxyethyl)-2-pyrazolio]methyl-3-cephem-4-carboxylate (syn isomer).
IR (Nujol) : 1765, 1590, 1530 $cm^{-1}$
NMR (DMSO-$d_6$, δ): 2.93 and 3.20 (2H, ABq, J = 17.0Hz), 3.59 (2H, m), 4.25-4.30 (2H, m), 4.40-4.60 (3H, m), 4.75-4.80 (1H, m), 5.02 (1H, d, J = 4.8Hz), 5.04 and 5.23 (2H, ABq, J = 15.0Hz), 5.65 (1H, dd, J = 8.4Hz, 4.8Hz), 5.82 (1H, d, J = 3.0Hz), 7.30 (2H, br s), 8.10 (1H, d, J = 3.0Hz), 8.19 (2H, br s), 9.57 (1H, d, J = 8.4Hz)

(4) 7β-[2-(5-Amino-1,2,4-thiadiazol-3-yl)-2-(2-fluoroethoxyimino)acetamido]-3-[2,3-dihydro-5-(1H-imidazo-[1,2-b]pyrazolio)]methyl-3-cephem-4-carboxylate (syn isomer)
IR (Nujol) : 1760, 1600, 1520 $cm^{-1}$
NMR (DMSO-$d_6$, δ) : 3.12 and 3.33 (2H, ABq, J = 17.0Hz), 3.95-4.05 (2H, m), 4.15-4.25 (2H, m), 4.40-4.55 (3H, m), 4.75-4.80 (1H, m), 4.98 and 5.08 (2H, ABq, J = 15.5Hz), 5.02 (1H, d, J = 4.8Hz), 5.64 (1H, dd, J = 4.8Hz, 8.4Hz), 5.87 (1H, d, J = 3.0Hz), 7.94 (1H, br s), 8.20 (2H, br s), 8.23 (1H, d, J = 3.0Hz), 9.55 (1H, d, J = 8.4Hz)

(5) 7β-[2-(5-Amino-1,2,4-thiadiazol-3-yl)-2-(2-fluoroethoxyimino)acetamido]-3-(4,5,6,7-tetrahydro-1-pyrazolo[1,5-a]pyrimidinio)methyl-3-cephem-4-carboxylate (syn isomer)
IR (Nujol) : 1775, 1620, 1530 $cm^{-1}$
NMR (DMSO-$d_6$, δ) : 2.03 (2H, br s), 3.04 and 3.20 (2H, ABq, J = 17.0Hz), 4.00-4.25 (4H, m), 4.25-4.30 (1H, m), 4.40-4.45 (1H, m), 4.50-4.55 (1H, m), 4.75-4.80 (1H, m), 5.01 (1H, d, J = 4.8Hz), 4.94 and 5.29 (2H, ABq, J = 15.1Hz), 5.63 (1H, dd, J = 4.8Hz, 8.4Hz), 5.80 (1H, d, J = 3.0Hz), 8.06 (1H, br s), 8.08 (1H, d,

J = 3.0Hz), 8.19 (2H, br s), 9.55 (1H, d, J = 8.4Hz)

(6) 7β-[2-(5-Amino-1,2,4-thiadiazol-3-yl)-2-fluoromethoxyiminoacetamido]-3-(4,5,6,7-tetrahydro-1-pyrazolo-[1,5-a]pyrimidinio)methyl-3-cephem-4-carboxylate (syn isomer)

IR (Nujol) : 1770, 1620, 1540 $cm^{-1}$

NMR (DMSO-$d_6$, δ) : 2.03 (2H, m), 3.05 and 3.21 (2H, ABq, J = 17.0Hz), 3.26 (2H, m), 4.15 (2H, m), 4.97 and 5.28 (2H, ABq, J = 15.0Hz), 5.03 (1H, d, J = 4.8Hz), 5.64 (1H, dd, J = 4.8Hz, 8.4Hz), 5.76 (2H, d, J = 50.0Hz), 5.80 (1H, d, J = 3.0Hz), 8.03 (1H, br s), 8.08 (1H, d, J = 3.0Hz), 8.25 (1H, br s), 9.69 (1H, d, J = 8.4Hz)

Example 2

A mixture of N,N-dimethylformamide (328 ml) and phosphorus oxychloride (400 ml) in ethyl acetate (8.5 ml) was stirred for 0.5 hour at 5 ° C. 2-(5-Tritylamino-1,2,4-thiadiazol-3-yl)-2-difluoromethoxyiminoacetic acid (syn isomer) (850 mg) was added thereto with stirring under ice-cooling, and the mixture was stirred for 30 minutes at 5 ° C. On the other hand, 7β-amino-3-[2,3-dihydro-5-(1H-imidazo[1,2-b]pyrazolio)]methyl-3-cephem-4-carboxylate hydrochloride (836 mg) was dissolved in a solution of N-(trimethylsilyl)acetamide (2.8 g) in N,N-dimethylformamide (8 ml) and tetrahydrofuran (8 ml). To the solution was added the solution obtained above at 0 ° C and the mixture was stirred for 1.5 hours at 5 ° C. The reaction mixture was poured into a mixture of ethyl acetate (50 ml) and water (50 ml). The organic layer was separated, washed with water and brine, dried over magnesium sulfate, and then evaporated in vacuo. The residue was triturated with diisopropyl ether to give 7β-[2-(5-tritylamino-1,2,4-thiadiazol-3-yl)-2-difluoromethoxyiminoacetamido]-3-[2,3-dihydro-5-(1H-imidazo[1,2-b]pyrazolio)]methyl-3-cephem-4-carboxylate (syn isomer) (1.65 g).

NMR (DMSO-$d_6$, δ) : 3.42 (2H, br s), 4.03-4.08 (2H, m), 4.09-4.15 (2H, m), 5.11 (2H, br s), 5.18 (1H, d, J = 5Hz), 5.85 (1H, dd, J = 5Hz, 8Hz), 5.94 (1H, d, J = 3Hz), 7.12 (1H, t, J = 70Hz), 7.10-7.23 (15H, m), 8.15 (1H, d, J = 3Hz), 9.86 (1H, d, J = 8Hz), 10.18 (1H, br s)

Example 3

To a solution of 7β-[2-5-tritylamino-1,2,4-thiadiazol-3-yl)-2-difluoromethoxyiminoacetamido]-3-(2,3-dihydro-5-(1H-imidazo[1,2-b]pyrazolio)]methyl-3-cephem-4-carboxylate (syn isomer) (0.25 g) in methanol (1.25 ml) was added conc. hydrochloric acid (0.17 ml) under stirring. The solution was stirred at 30 ° C for 2 hours. A mixture of ethyl acetate (30 ml) and water (30 ml) was added thereto and then adjusted to pH 3.0 with 1N hydrochloric acid. The separated aqueous layer was washed with ethyl acetate (20 ml) and subjected to column chromatography on Diaion HP-20 using 10% aqueous isopropyl alcohol as an eluent and then the object fractions were lyophilized to give 7β-[2-(5-amino-1,2,4-thiadiazol-3-yl)-2-difluoromethoxyimino)acetamido]-3-[2,3-dihydro-5-(1H-imidazo[1,2-b]pyrazolio)]methyl-3-cephem-4-carboxylate (syn isomer) (150 mg).

IR (Nujol) : 1760, 1660, 1600, 1520 $cm^{-1}$

NMR ($D_2O$, δ): 3.30 and 3.48 (2H, ABq, J = 18Hz), 4.13-4.40 (4H, m), 4.80-5.08 (2H, m), 5.26 (1H, d, J = 5Hz), 5.85 (1H, d, J = 3Hz), 5.88 (1H, d, J = 5Hz), 6.99 (1H, t, J = 70Hz), 7.95 (1H, d, J = 3Hz)

Example 4

(1) A solution of phosphorus pentachloride (686 mg) in dichloromethane (6 ml) was stirred at room temperature for 20 minutes. 2-(5-Amino-1,2,4-thiadiazol-3-yl)-2-(2,2,2-trifluoroethoxyimino)acetic acid (syn isomer) (810 mg) was added to the solution at -10 ° C with stirring. After stirred at the same temperature for one hour, the solvent was evaporated in vacuo under ice-cooling to give 2-(5-amino-1,2,4-thiadiazol-3-yl)-2-(2,2,2-trifluoroethoxyimino)acetyl chloride (syn isomer) (Compound I). On the other hand, to a solution of 7β-amino-3-[1-(2-hydroxyethyl)-5-amino-2-pyrazolio]methyl-3-cephem-4-carboxylate hydrochloride (1030 mg) in a mixture of tetrahydrofuran (10.3 ml) and N,N-dimethylformamide (10.3 ml) was added N-(trimethylsilyl)acetamide (4.92 g). After stirred at 35 ° C for 15 minutes, the solution was cooled to 5 ° C. To the cool solution was added a tetrahydrofuran (6 ml) solution of the compound I obtained above. After stirred at ice-cooling for 1 hour, the solution was poured into ethyl acetate (400 ml) and triturated to give a crude product. The crude product was subjected to column chromatography on acidic alumina (12 ml) with pH 4.0 (eluent : water) and on Diaion HP-20 (55 ml) with pH 3.0 (eluent, 10% aqueous isopropyl alcohol). The eluate was evaporated in vacuo and freeze-dried to give 7β-[2-(5-amino-1,2,4-thiadiazol-3-yl)-2-(2,2,2-trifluoroethoxyimino)acetamido]-3-[1-(2-hydroxyethyl)-5-amino-2-pyrazolio]-methyl-3-cephem-4-carboxylate (syn isomer) (610 mg).

IR (Nujol) : 1760, 1590, 1530 $cm^{-1}$
NMR (DMSO-$d_6$, δ) : 2.91 and 3.20 (2H, ABq, J = 17.1Hz), 3.58 (2H, m), 4.20-4.60 (2H, m), 4.60-4.90 (2H, m), 5.02 (1H, d, J = 4.8Hz), 5.05 and 5.22 (2H, ABq, J = 15.2Hz), 5.64 (1H, dd, J = 8.4Hz, 4.8Hz), 5.81 (1H, d, J = 3.1Hz), 7.30 (2H, br s), 8.10 (1H, d, J = 3.1Hz), 8.22 (2H, br s), 9.70 (1H, d, J = 8.4Hz)

The following compounds were obtained according to a similar manner to that of Example 4-(1).

(2) 7β-[2-(5-Amino-1,2,4-thiadiazol-3-yl)-2-(2,2,2-trifluoroethoxyimino)acetamido]-3-[2,3-dihydro-5-(1H-imidazo[1,2-b]pyrazolio)]methyl-3-cephem-4-carboxylate (syn isomer)
IR (Nujol) : 1760, 1600, 1520 $cm^{-1}$
NMR (DMSO-$d_6$, δ) : 3.10 and 3.34 (2H, ABq, J = 17.1Hz), 3.85-4.10 (2H, m), 4.10-4.40 (1H, m), 4.40-4.60 (1H, m), 4.65-4.90 (2H, m), 4.97 and 5.08 (2H, ABq, J = 15.1Hz), 5.02 (1H, d, J = 4.8Hz), 5.63 (1H, dd, J = 8.4Hz, 4.8Hz), 5.87 (1H, d, J = 3.0Hz), 7.91 (1H, br s), 8.23 (2H, br s), 8.24 (1H, d, J = 3.0Hz), 9.65 (1H, d, J = 8.4Hz)

(3) 7β-[2-(5-Amino-1,2,4-thiadiazol-3-yl)-2-(2,2,2-trifluoroethoxyimino)acetamido]-3-(4,5,6,7-tetrahydro-1-pyrazolo[1,5-a]pyrimidinio)methyl-3-cephem-4-carboxylate (syn isomer)
IR (Nujol) : 1775, 1620, 1540 $cm^{-1}$
NMR (DMSO-$d_6$, δ) : 2.03 (2H, m), 3.02 and 3.20 (2H, ABq, J = 17.1Hz), 3.25 (2H, m), 4.14 (2H, m), 4.65-4.95 (2H, m), 5.00 (1H, d, J = 4.8Hz), 4.95 and 5.28 (2H, ABq, J = 15.1Hz), 5.62 (1H, dd, J = 4.8Hz, 8.4Hz), 5.80 (1H, d, J = 3.2Hz), 8.01 (1H, br s), 8.08 (1H, d, J = 3.2Hz), 8.23 (2H, br s), 9.65 (1H, d, J = 8.4Hz)

## Example 5

The following compounds were obtained according to similar manners to those of Example 1-(1) and Example 4-(1).

(1) 7β-[2-(5-Amino-1,2,4-thiadiazol-3-yl)-2-(2,2-difluoroethoxyimino)acetamido]-3-[1-(2-hydroxyethyl)-5-amino-2-pyrazolio]methyl-3-cephem-4-carboxylate (syn isomer)
IR (Nujol) : 1760, 1600, 1520 $cm^{-1}$
NMR ($D_2O$, δ) : 3.13 and 3.38 (2H, ABq, J = 17.8Hz), 3.86 (2H, m), 4.35 (2H, m), 4.56 (2H, dt, J = 3.6Hz, 14.3Hz), 4.95 and 5.12 (2H, ABq, J = 14.8Hz), 5.20 (1H, d, J = 4.7Hz), 5.86 (1H, d, J = 4.7Hz), 5.98 (1H, d, J = 3.3Hz), 6.20 (1H, tt, J = 3.6Hz, 54.4Hz), 7.91 (1H, d, J = 3.3Hz)

(2) 7β-[2-(5-Amino-1,2,4-thiadiazol-3-yl)-2-(2,2-difluoroethoxyimino)acetamido]-3-[2,3-dihydro-5-(1H-imidazo[1,2-b]pyrazolio)]methyl-3-cephem-4-carboxylate (syn isomer)
IR (Nujol) : 1760, 1600, 1520 $cm^{-1}$
NMR ($D_2O$, δ) : 3.27 and 3.50 (2H, ABq, J = 17.7Hz), 4.10-4.40 (4H, m), 4.56 (2H, dt, J = 3.6Hz, 14.3Hz), 4.95 and 5.13 (2H, ABq, J = 15.4Hz), 5.24 (1H, d, J = 4.8Hz), 5.86 (1H, d, J = 3.2Hz), 5.88 (1H, d, J = 4.8Hz), 6.20 (1H, tt, J = 3.6Hz, 54.4Hz), 7.95 (1H, d, J = 3.2Hz)

(3) 7β-[2-(5-Amino-1,2,4-thiadiazol-3-yl)-2-(2,2-difluoroethoxyimino)acetamido]-3-(4,5,6,7-tetrahydro-1-pyrazolo[1,5-a]pyrimidinio)methyl-3-cephem-4-carboxylate (syn isomer)
IR (Nujol) : 1760, 1610, 1520 $cm^{-1}$
NMR ($D_2O$, δ) : 2.13 (2H, m), 3.19 and 3.34 (2H, ABq, J = 17.7Hz), 3.35 (2H, m), 4.07 (2H, m), 4.55 (2H, dt, J = 3.6Hz, 14.3Hz), 4.90 and 5.25 (2H, ABq, J = 16.1Hz), 5.22 (1H, d, J = 4.8Hz), 5.81 (1H, d, J = 3.4Hz), 5.90 (1H, d, J = 4.8Hz), 6.20 (1H, tt, J = 3.6Hz, 54.4Hz), 7.78 (1H, d, J = 3.4Hz)

(4) 7β-[2-(5-Amino-1,2,4-thiadiazol-3-yl)-2-(2,2,3,3,3-pentafluoropropoxyimino)acetamido]-3-[1-(2-hydroxyethyl)-5-amino-2-pyrazolio]methyl-3-cephem-4-carboxylate (syn isomer)
IR (Nujol) : 3300, 3170, 1770, 1600 $cm^{-1}$
NMR (DMSO-$d_6$, δ) : 2.92 and 3.19 (2H, ABq, J = 17.2Hz), 3.59 (2H, m), 4.2-4.5 (2H, m), 4.7-5.0 (2H, m), 5.02 (1H, d, J = 4.8Hz), 5.04 and 5.21 (2H, ABq, J = 15.8Hz), 5.63 (1H, dd, J = 4.8Hz, 8.4Hz), 5.81 (1H, d, J = 3.2Hz), 7.29 (2H, br s), 8.09 (1H, d, J = 3.2Hz), 8.23 (2H, br s), 9.65 (1H, d, J = 8.4Hz)

(5) 7β-[2-(5-Amino-1,2,4-thiadiazol-3-yl)-2-(2,2,3,3,3-pentafluoropropoxyimino)acetamido]-3-[2,3-dihydro-5-(1H-imidazo[1,2-b]pyrazolio)]methyl-3-cephem-4-carboxylate (syn isomer)
IR (Nujol) : 3300, 1770, 1610 $cm^{-1}$
NMR (DMSO-$d_6$, δ) : 3.12 and 3.33 (2H, ABq, J = 17.2Hz), 4.01 (2H, t, J = 7.7Hz), 4.23 (1H, t, J = 8.5Hz), 4.48 (1H, t, J = 8.1Hz), 4.7-5.0 (2H, m), 5.01 (1H, d, J = 4.8Hz), 4.99 and 5.08 (2H, ABq, J = 15.5Hz), 5.64 (1H, dd, J = 4.8Hz, 8.4Hz), 5.87 (1H, d, J = 3.2Hz), 7.96 (1H, br s), 8.23 (1H, d, J = 3.2Hz), 8.25 (2H, br s), 9.63 (1H, d, J = 8.4Hz)

## Example 6

To a solution of 7β-[2-(5-amino-1,2,4-thiadiazol-3-yl)-2-(2,2-difluoroethoxyimino)acetamido]-3-(1-(2-hydroxyethyl)-5-amino-2-pyrazolio]methyl-3-cephem-4-carboxylate (syn isomer) (2 g) in 0.1 mole - sulfuric acid (34.9 ml). The solution was lyophilized to give 7β-[2-(5-amino-1,2,4-thiadiazol-3-yl)-2-(2,2-difluoroethoxyimino)acetamido]-3-(1-(2-hydroxyethyl-5-amino-2-pyrazolio]methyl-3-cephem-4-carboxylate sulfate (syn isomer) (2.34 g).
IR (Nujol): 1770, 1660, 1600, 1520 $cm^{-1}$
NMR (DMSO-$d_6$, δ): 3.20 and 3.33 (2H, ABq, J = 18Hz), 3.61 (2H, br s), 3.98-4.60 (2H + 2H, m), 5.10 and 5.31 (2H, ABq, J = 16Hz), 5.20 (1H, d, J = 5Hz), 5.90 (1H, dd, J = 5Hz, 8Hz), 5.92 (1H, d, J = 3Hz), 6.20 (1H, tt, J = 3.6Hz, 54.6Hz), 7.32 (2H, br s), 7.98 (1H, d, J = 3Hz), 8.19 (2H, br s), 9.69 (1H, d, J = 8Hz)

Example 7

To a solution of 7β-[2-(5-amino-1,2,4-thiadiazol-3-yl)-2-(2,2-difluoroethoxyimino)acetamido]-3-[1-(2-hydroxyethyl)-5-amino-2-pyrazolio]methyl-3-cephem-4-carboxylate (syn isomer) (2 g) in 4N-hydrochloric acid (2 ml) was added acetonitrile (8 ml) under stirring at 20 °C. The stirring was continued for 2 hours at the same temperature to give crystals, which were collected by filtration and dried to give 7β-[2-(5-amino-1,2,4-thiadiazol-3-yl)-2-(2,2-difluoroethoxyimino)acetamido]-3-[1-(2-hydroxyethyl)-5-amino-2-pyrazolio]-methyl-3-cephem-4-carboxylate hydrochloride (syn isomer) as crystals (1.4 g).
IR (Nujol): 3230, 3130, 1780, 1705, 1630, 1605 $cm^{-1}$
NMR (DMSO-$d_6$, δ): 3.25 and 3.35 (2H, ABq, J = 18Hz), 3.60 (2H, br s), 4.00-4.62 (2H + 2H, m), 5.14 and 5.32 (2H, ABq, J = 16Hz), 5.18 (1H, d, J = 5Hz), 5.90 (1H, dd, J = 5HZ, 8Hz), 5.91 (1H, d, J = 3Hz), 6.20 (1H, tt, J = 3.6Hz, 54.6Hz), 7.49 (2H, br s), 8.02 (1H, d, J = 3Hz), 8.27 (2H, br s), 9.78 (1H, d, J = 8Hz)

Example 8

To a mixture of tetrahydrofuran (2.5 ml) and N,N-dimethylformamide (0.78 ml), phosphorus oxychloride (0.85 ml) was added at 5 ° C. After stirred at 5 ° C for 15 minutes, tetrahydrofuran (22 ml) and then 2-(5-triphenylmethylamino-1,2,4-thiadiazol-3-yl)-2-difluoromethoxyiminoacetic acid (syn isomer) (2.21 g) were added thereto, and the resulting solution was stirred at 5 ° C for 30 minutes. The solution was added to a solution of 7β-amino-3-[1-(2-hydroxyethyl)-5-amino-2-pyrazolio]methyl-3-cephem-4-carboxylate hydrochloride dihydrate (2.08 g) and N-(trimethylsilyl)acetamide (9.96 g) in tetrahydrofuran (31 ml) at 7 ° C. After stirred at 5 ° C for 60 minutes, the mixture was poured into a mixture of ethyl acetate (200 ml), tetrahydrofuran (100 ml) and water (150 ml), and adjusted to pH 7 with a saturated aqueous sodium hydrogencarbonate solution. The separated organic phase was washed with brine (150 ml) and dried with magnesium sulfate and evaporated. A solution of the residue in tetrahydrofuran (10 ml) was added dropwise to isopropyl ether (80 ml), and the resulting powder was collected by filtration and dried in vacuo to give 7β-[2-(5-triphenylmethylamino-1,2,4-thiadiazol-3-yl)-2-difluoromethoxyiminoacetamido]-3-[1-(2-hydroxyethyl)-5-amino-2-pyrazolio]methyl-3-cephem-4-carboxylate (syn isomer) (1.07 g).
IR (Nujol) : 1770 $cm^{-1}$
NMR (DMSO-$d_6$, δ) : 2.91 and 3.20 (2H, ABq, J = 17.0Hz), 3.60 (2H, m), 4.25-4.30 (2H, m), 5.02 (1H, d, J = 4.9Hz), 5.06 and 5.22 (2H, ABq, J = 19.5Hz), 5.58 (1H, dd, J = 4.9Hz, 8.1Hz), 5.81 (1H, d, J = 3.2Hz), 7.18 (1H, t, J = 70.4Hz), 7.20-7.45 (15H, m), 8.09 (1H, d, J = 3.2Hz), 9.79 (1H, d, J = 8.1Hz), 10.16 (1H, s)

Example 9

The following compound was obtained according to a similar manner to that of Example 8.
7β-[2-(5-Triphenylmethylamino-1,2,4-thiadiazol-3-yl)-2-difluoromethoxyiminoacetamido]-3-(4,5,6,7-tetrahydro-1-pyrazolo[1,5-a]pyrimidinio)methyl-3-cephem-4-carboxylate (syn isomer)
IR (Nujol) : 1770 $cm^{-1}$
NMR (DMSO-$d_6$, δ) : 1.90-2.10 (2H, m), 3.10-3.60 (4H, m), 4.08 (2H, m), 4.98 and 5.29 (2H, ABq, J = 19.5Hz), 5.04 (1H, d, J = 4.8Hz), 5.63 (1H, dd, J = 4.8Hz, 8.0Hz), 5.81 (1H, d, J = 3.3Hz), 7.18 (1H, t, J = 70.1Hz), 7.18-7.37 (15Hz, m), 8.04 (1H, d, 3.3Hz), 9.79 (1H, d, J = 8.0Hz), 10.17 (1H, s)

Example 10

To a mixture of trifluoroacetic acid (15 ml) and anisole (9.2 ml), 7β-[2-(5-triphenylmethylamino-1,2,4-thiadiazol-3-yl)-2-difluoromethoxyiminoacetamido]-3-[1-(2-hydroxyethyl)-5-amino-2-pyrazolio]methyl-3-cephem-4-carboxylate (syn isomer) (1.06 g) was added at 5 ° C. After stirred at room temperature for 2.5 hours, the resulting solution was poured into isopropyl ether (120 ml). The resulting powder was collected

by filtration, and dried in vacuo. The powder was dissolved in a mixture of ethyl acetate (60 ml) and water (60 ml) by adjustment to pH 7 with a saturated aqueous sodium hydrogencarbonate solution. The insoluble residue was removed off. After adjusted to pH 4 with 6N-hydrochloric acid, the separated water phase was evaporated to remove the containing organic solvent. The water solution was subjected to column chromatography on ICN Alumina A Super I (Trademark: ICN Biochemicals) (6.4 g), and eluted with water. The fractions containing the object compound were collected. After adjusted to pH 4 with 6N-hydrochloric acid, the collected water solution was subjected to column chromatography on HP-20 (Trademark: Mitsubishi Kasei Corporation) (64 ml). The column was washed with water (190 ml) and the object compound was eluted with 30% aqueous methanol, and the eluate was lyophilized to give 7β-[2-(5-amino-1,2,4-thiadiazol-3-yl)-2-difluoromethoxyiminoacetamido]-3-[1-(2-hydroxyethyl)-5-amino-2-pyrazolio]methyl-3-cephem-4-carboxylate (syn isomer) (1.28 g).

IR (Nujol) : 1770 $cm^{-1}$

NMR ($D_2O$, δ) : 3.13 and 3.40 (2H, ABq, J=18.7Hz), 3.88 (2H, m), 4.33 (2H, m), 5.05 and 5.23 (2H, ABq, J=15.2Hz), 5.25 (1H, d, J=4.8Hz), 5.88 (1H, d, J=4.8Hz), 5.97 (1H, d, J=3.3Hz), 6.99 (1H, t, J=69.8Hz), 7.90 (1H, d, J=3.3Hz)

Example 11

The following compound was obtained according to a similar manner to that of Example 10.

7β-[2-(5-Amino-1,2,4-thiadiazol-3-yl)-2-difluoromethoxyiminoacetamido]-3-(4,5,6,7-tetrahydro-1-pyrazolo-[1,5-a]pyrimidinio)methyl-3-cephem-4-carboxylate (syn isomer)

IR (Nujol) : 1770 $cm^{-1}$

NMR ($D_2O$, δ) : 2.13 (2H, m), 3.15-3.50 (4H, m), 4.02-4.14 (2H, m), 4.91 and 5.25 (2H, ABq, J=15.9Hz), 5.23 (1H, d, J=4.8Hz), 5.82 (1H, d, J=3.4Hz), 5.89 (1H, d, J=4.8Hz), 6.99 (1H, t, J=69.8Hz), 7.78 (1H, d, J=3.4Hz)

Example 12

A solution of 7β-amino-3-[1-(2-hydroxyethyl)-5-amino-2-pyrazolio]methyl-3-cephem-4-carboxylate hydrochloride (3.1 g) in a mixture of acetonitrile (30 ml) and water (15 ml) was adjusted to pH 6.0 with an aqueous sodium hydrogencarbonate solution. To the resulting solution was added methanesulfonic 2-(5-amino-1,2,4-thiadiazol-3-yl)-2-(2,2-difluoroethoxyimino)acetic anhydride (syn isomer (3 g) under stirring at 5°C, and then the stirring was continued at the same temperature for 1.5 hours under adjusting to pH 5.0~6.5 with an aqueous sodium hydrogencarbonate. The reaction mixture was evaporated to remove the organic solvent. The aqueous solution was subjected to column chromatography on SP-205 (trademark : Mitsubishi Kasei Corporation) (75 ml). The column was washed with water (400 ml) and the object compound was eluted with 15% aqueous 2-propanol, and the eluate was lyophilized to give 7β-[2-(5-amino-1,2,4-thiadiazol-3-yl)-2-(2,2-difluoroethoxyimino)acetamido]-3-[1-(2-hydroxyethyl)-5-amino-2-pyrazolio]-methyl-3-cephem-4-carboxylate (syn isomer) (3.1 g).

NMR ($D_2O$, δ) : 3.13 and 3.38 (2H, ABq, J=17.8Hz), 3.86 (2H, m), 4.35 (2H, m), 4.56 (2H, dt, J=3.6Hz, 14.3Hz), 4.95 and 5.12 (2H, ABq, J=14.8Hz), 5.20 (1H, d, J=4.7Hz), 5.86 (1H, d, J=4.7Hz), 5.98 (1H, d, J=3.3Hz), 6.20 (1H, tt, J=3.6Hz, J=54.4Hz), 7.91 (1H, d, J=3.3Hz)

Example 13

To a suspension of 7β-amino-3-[1-(2-fluoroethyl)-5-amino-2-pyrazolio]methyl-3-cephem-4-carboxylate hydrochloride (756 mg) in a mixture of tetrahydrofuran (7.6 ml) and N,N-dimethylformamide (7.6 ml) was added N-(trimethylsilyl)acetamide (3.94 g). After stirred at 35°C for 15 minutes, the mixture was turned to clear. The resulting solution was cooled to 5°C, and then methanesulfonic 2-(5-amino-1,2,4-thiadiazol-3-yl)-2-(2,2-difluoroethoxyimino)acetic anhydride (syn isomer) (793 mg) was added to the cold solution. After stirred under ice-cooling for 1 hour, the solution was poured into ethyl acetate (400 ml). The resulting precipitates were collected by filtration and dried in vacuo. The precipitates were dissolved into water, adjusted to pH 4.0 with an aqueous sodium hydrogencarbonate solution and then subjected to column chromatography on acidic alumina (9 ml) [eluent : water]. The eluate was adjusted to pH 3.0 with 1N-hydrochloric acid and subjected to column chromatography on Diaion HP-20 (45 ml) [eluent; 10% aqueous isopropylalcohol]. The eluate was evaporated in vacuo and freeze-dried to give 7β-[2-(5-amino-1,2,4-thiadiazol-3-yl)-2-(2,2-difluoroethoxyimino)acetamido]-3-[1-(2-fluoroethyl)-5-amino-2-pyrazolio]methyl-3-cephem-4-carboxylate (syn isomer) (840 mg).

IR (Nujol) : 3400, 3200, 1760, 1680, 1600 $cm^{-1}$
NMR ($D_2O$, $\delta$) : 3.12 and 3.41 (2H, ABq, J=17.8Hz), 4.4-5.0 (4H, m), 4.56 (2H, dt, J=3.6Hz, J=14.3Hz), 5.07 and 5.17 (2H, ABq, J=14.8Hz), 5.23 (1H, d, J=4.7Hz), 5.83 (1H, d, J=4.7Hz), 5.97 (1H, d, J=3.3Hz), 6.19 (1H, tt, J=3.6Hz, J=54.4Hz), 7.92 (1H, d, J=3.3Hz)

## Claims

**1.** A compound of the formula :

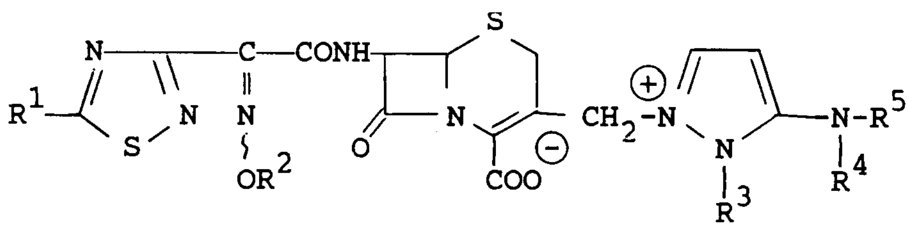

wherein

| | |
|---|---|
| $R^1$ is | amino or protected amino, |
| $R^2$ is | lower alkyl substituted with 1 to 6 halogen, |
| $R^3$ is | hydroxy(lower)alkyl, protected hydroxy(lower)alkyl or halo(lower)alkyl and |
| $R^4$ is | hydrogen, or |
| $R^3$ and $R^4$ are | linked together to form lower alkylene, and |
| $R^5$ is | hydrogen or an amino protective group, |

and a pharmaceutically acceptable salt thereof.

**2.** A compound of claim 1, wherein

| | |
|---|---|
| $R^1$ is | amino or amino substituted by ar(lower)alkyl which may have suitable substituent(s), |
| $R^2$ is | lower alkyl substituted with 1 to 6 fluorine, |
| $R^3$ is | hydroxy(lower)alkyl or fluoro(lower)alkyl and |
| $R^4$ is | hydrogen, or |
| $R^3$ and $R^4$ are | linked together to form lower alkylene, and |
| $R^5$ is | hydrogen. |

**3.** A compound of claim 2, wherein

| | |
|---|---|
| $R^1$ is | amino or amino substituted by mono(or di or tri)phenyl(lower)alkyl, |
| $R^2$ is | ($C_1$-$C_4$)alkyl substituted by 1 to 5 fluorine, |
| $R^3$ is | hydroxy($C_1$-$C_4$)alkyl or fluoro($C_1$-$C_4$)alkyl and |
| $R^4$ is | hydrogen, or |
| $R^3$ and $R^4$ are | linked together to form ($C_1$-$C_4$)alkylene, and |
| $R^5$ is | hydrogen. |

**4.** A compound of claim 3, wherein

| | |
|---|---|
| $R^1$ is | amino or tritylamino, and |
| $R^2$ is | ($C_1$-$C_3$)alkyl substituted with 1 to 5 fluorine. |

**5.** A compound of claim 4, wherein

| | |
|---|---|
| $R^1$ is | amino, |
| $R^2$ is | mono(or di)fluoro($C_1$-$C_3$)alkyl, |
| $R^3$ is | hydroxy($C_1$-$C_4$)alkyl or fluoro($C_1$-$C_4$)alkyl, |
| $R^4$ is | hydrogen, and |
| $R^5$ is | hydrogen. |

**6.** A compound of claim 5, which is selected from the group consisting of

(1) 7β-[2-(5-Amino-1,2,4-thiadiazol-3-yl)-2-fluoromethoxyiminoacetamido]-3-[5-amino-1-(2-hydroxyethyl)-2-pyrazolio]methyl-3-cephem-4-carboxylate (syn isomer),

(2) 7β-[2-(5-Amino-1,2,4-thiadiazol-3-yl)-2-(2-fluoroethoxyimino)acetamido]-3-[5-amino-1-(2-hydroxyethyl)-2-pyrazolio]methyl-3-cephem-4-carboxylate (syn isomer),
(3) 7β-[2-(5-Amino-1,2,4-thiadiazol-3-yl)-2-(2,2-difluoroethoxyimino)acetamido]-3-[1-(2-hydroxyethyl)-5-amino-2-pyrazolio]methyl-3-cephem-4-carboxylate hydrochloride (syn isomer) and
(4) 7β-[2-(5-Amino-1,2,4-thiadiazol-3-yl)-2-(2,2-difluoroethoxyimino)acetamido]-3-[1-(2-fluoroethyl)-5-amino-2-pyrazolio]methyl-3-cephem-4-carboxylate (syn isomer).

**7.** A process for preparing a compound of the formula :

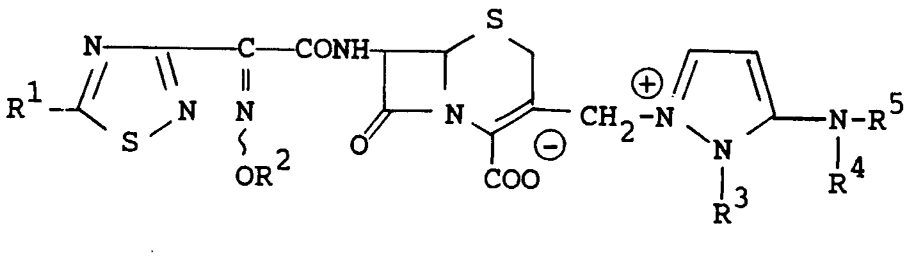

wherein
$R^1$ is amino or protected amino,
$R^2$ is lower alkyl substituted with 1 to 6 halogen,
$R^3$ is hydroxy(lower)alkyl, protected hydroxy(lower)alkyl or halo(lower)alkyl and
$R^4$ is hydrogen, or
$R^3$ and $R^4$ are linked together to form lower alkylene, and
$R^5$ is hydrogen or an amino protective group,
or a salt thereof,
which comprises
(1) reacting a compound of the formula :

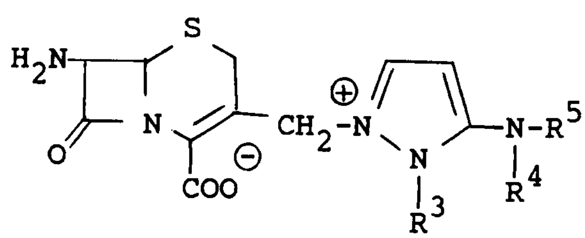

wherein $R^3$, $R^4$ and $R^5$ are each as defined above,
or its reactive derivative at the amino group,
or a salt thereof with a compound of the formula :

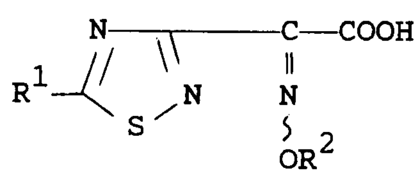

wherein $R^1$ and $R^2$ are each as defined above,
or its reactive derivative at the carboxy group,
or a salt thereof to give a compound of the formula :

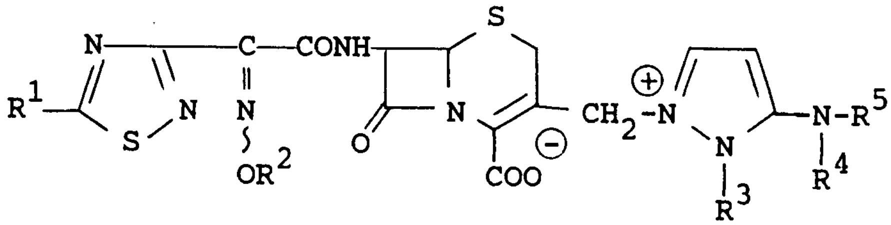

wherein

$R^1$, $R^2$, $R^3$, $R^4$ and $R^5$ are each as defined above,

or a salt thereof, or

(2) subjecting a compound of the formula :

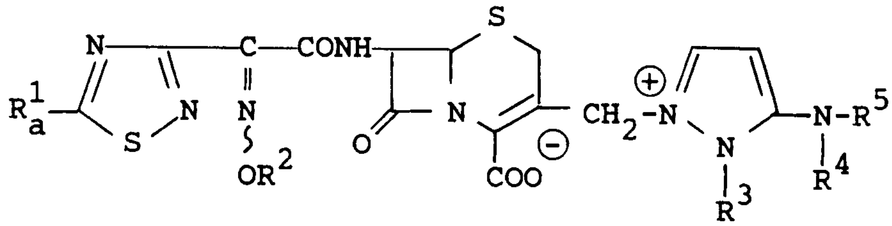

wherein

$R^2$, $R^3$, $R^4$ and $R^5$ are each as defined above, and

$R^1_a$ is protected amino,

or a salt thereof to elimination reaction of the amino protective group to give a compound of the formula :

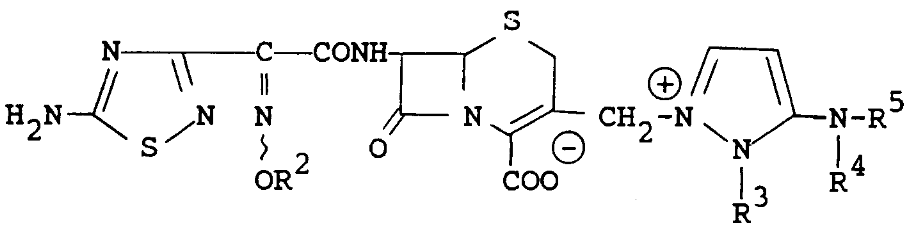

wherein

$R^2$, $R^3$, $R^4$, $R^5$ are each as defined above,

or a salt thereof.

**8.** A compound of the formula :

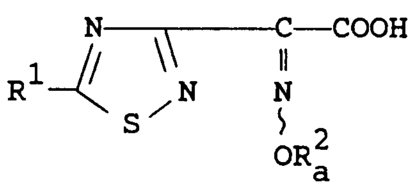

wherein

$R^1$ is amino or protected amino, and

$R^2_a$ is mono(or di)fluoroethyl or pentafluoro(lower)alkyl,

or its reactive derivative at the carboxy group, or a salt thereof.

**9.** A process for preparing a compound of the formula :

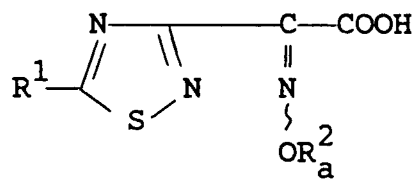

wherein
$R^1$ is amino or protected amino, and
$R_a^2$ is mono(or di)fluoroethyl or pentafluoro(lower)alkyl,
or a salt thereof,
which comprises
(1) reacting a compound of the formula :

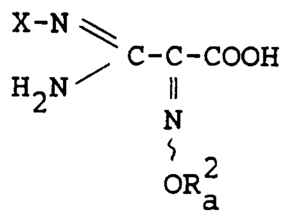

wherein
$R_a^2$ is as defined above, and
X is halogen,
or a salt thereof with a compound of the formula :

M S C N

wherein M is alkali metal to give a compound of the formula :

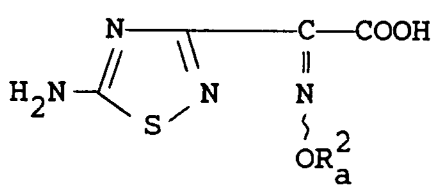

wherein $R_a^2$ is as defined above,
or a salt thereof, or
(2) subjecting a compound of the formula :

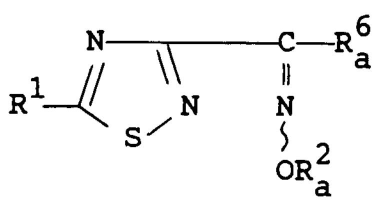

wherein
$R^1$ and $R_a^2$ are each as defined above, and
$R_a^6$ is protected carboxy,
or a salt thereof to elimination reaction of the carboxy protective group to give a compound of the formula :

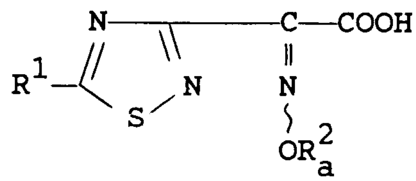

wherein $R^1$ and $R_a^2$ are each as defined above, or a salt thereof, or

(3) reacting a compound of the formula :

$$H_2N - OR_a^2$$

wherein $R_a^2$ is as defined above, or a salt thereof with a compound of the formula :

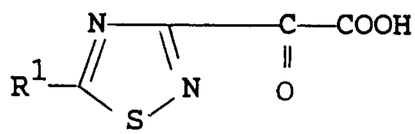

wherein $R^1$ is as defined above, or a salt thereof to give a compound of the formula :

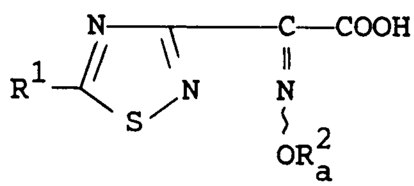

wherein $R^1$ and $R_a^2$ are each as defined above, or a salt thereof, or

(4) subjecting a compound of the formula :

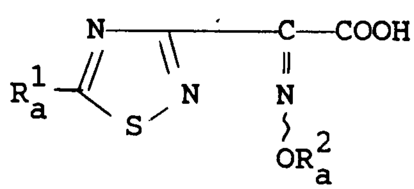

wherein

$R_a^2$ is as defined above, and

$R_a^1$ is protected amino,

or a salt thereof to elimination reaction of the amino protective group to give a compound of the formula :

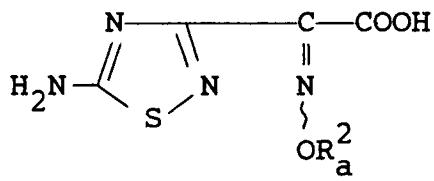

wherein
$R^2_a$ is as defined above,
or a salt thereof, or
(5) subjecting a compound of the formula :

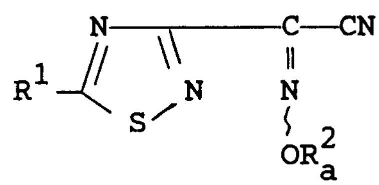

wherein
$R^1$ and $R^2_a$ are each as defined above,
or a salt thereof to hydrolysis to give a compound of the formula :

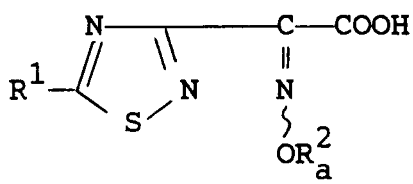

wherein
$R^1$ and $R^2_a$ are each as defined above,
or a salt thereof.

**10.** A pharmaceutical composition which comprises, as an active ingredient, a compound of claim 1 or a pharmaceutically acceptable salt thereof in admixture with pharmaceutically acceptable carriers.

**11.** Use of a compound of claim 1 or a pharmaceutically acceptable salt thereof for the manufacture of a medicament for treating or preventing infectious diseases.

**12.** A compound of claim 1 or a pharmaceutically acceptable salt thereof for use an an antimicrobial agent.

European Patent Office

# EUROPEAN SEARCH REPORT

Application Number

EP 92 10 8580

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 332 156 (FUJISAWA)<br>* Complete specification; page 8, lines 57-59 * | 1-12 | C 07 D 501/46<br>C 07 D 285/08<br>A 61 K 31/545 |
| Y | EP-A-0 223 246 (FUJISAWA)<br>* Complete specification * | 1-12 | |
| Y | EP-A-0 307 804 (FUJISAWA)<br>* Complete specification; page 10, lines 43-50; examples 3,5,6,8,9,24 * | 1-12 | |
| Y | EP-A-0 427 248 (FUJISAWA)<br>* Complete specification; page 10, lines 32-34 * | 1-12 | |
| | | | TECHNICAL FIELDS SEARCHED (Int. Cl.5) |
| | | | C 07 D 501/00<br>C 07 D 285/00 |

The present search report has been drawn up for all claims

| Place of search | Date of completion of the search | Examiner |
|---|---|---|
| THE HAGUE | 17-09-1992 | LUYTEN H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)